(19) Europäisches Patentamt
European Patent Office
Office européen des brevets

(11) **EP 4 481 106 A1**

(12) **EUROPEAN PATENT APPLICATION**

(43) Date of publication:
**25.12.2024 Bulletin 2024/52**

(21) Application number: **23305985.6**

(22) Date of filing: **21.06.2023**

(51) International Patent Classification (IPC):
***D06M 11/05*** *(2006.01)* ***A61L 27/52*** *(2006.01)*
***D01F 1/10*** *(2006.01)* ***D01F 6/14*** *(2006.01)*
***D06M 13/00*** *(2006.01)* ***D06M 15/00*** *(2006.01)*

(52) Cooperative Patent Classification (CPC):
(C-Sets available)
**D06M 13/00; A61L 27/16; A61L 27/50; A61L 27/52; D01F 6/14; D01F 8/10; D06M 11/05; D06M 15/00;**
A61L 27/507; A61L 27/60; A61L 2430/06; A61L 2430/10; A61L 2430/30; A61L 2430/32; A61L 2430/34; (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Ecole Nationale Superieure des Mines de Paris**
  **75272 Paris cedex 06 (FR)**
- **Association pour la Recherche et le Développement des Méthodes et Processus Industriels (ARMINES)**
  **75272 Paris Cedex 06 (FR)**

(72) Inventors:
- **CORTE, Laurent**
  **94400 Vitry-sur-Seine (FR)**
- **CAROUX, Julien**
  **91300 Massy (FR)**
- **MAEZTU REDIN, Juan Deyo**
  **75272 Paris cedex 06 (FR)**

(74) Representative: **Icosa**
**83 avenue Denfert-Rochereau**
**75014 Paris (FR)**

(54) **FIBER ASSEMBLY AND USE THEREOF AS PRESTRESSED HYDROGELS AND TISSUE SUBSTITUTES**

(57) The present invention relates to a fiber assembly comprising at least two fibers, the at least two fibers comprising a polymer consisting of at least two polymer chains, the at least two polymer chains being chemically or physically crosslinked with each other and oriented on a macromolecular scale in the dry state, the at least two fibers being nonwoven, twisted, woven, braided or knitted together, or included within a polymeric matrix further comprised in the fiber assembly.

EP 4 481 106 A1

(52) Cooperative Patent Classification (CPC): (Cont.)
A61L 2430/38

C-Sets
**A61L 27/16, C08L 29/04**

## Description

### FIELD OF INVENTION

The present invention relates to a fiber assembly comprising at least two fibers which undergo contraction when placed in contact with a swelling agent, the at least two fibers being either twisted, woven, non-woven, braided or knitted together, or included within a polymeric matrix further comprised in the fiber assembly. The present invention also relates to the use of said fiber assembly as implantable materials, as substitutes for the reconstruction, replacement or augmentation of tissues, as tissue-mimicking phantoms, as matrices for cell culture, or as scaffolds for tissue engineering. The present invention further relates to a process for manufacturing said fiber assembly.

### BACKGROUND OF INVENTION

Due to their similarities with the extra-cellular matrix and with biological tissues in general, hydrogels are of utmost interest in a great number of biological and medical applications. One could mention implantable substitutes for tissue reconstruction or replacement, scaffolds for tissue engineering, implants for controlled drug release, encapsulation matrices for cell therapies, tissue-mimicking phantoms for the development of biomechanical models and the calibration of imaging techniques or for the development of new treatments or for surgical training.

In these applications, it is essential that these hydrogel materials closely mimic the mechanical behavior of biological tissues. This is needed to restore the biomechanical function in the case of soft tissue substitutes, to apply biomimetic mechanical cues to stimulate the appropriate cellular activity in tissue engineering, and to reproduce the response of tissues to an imaging technique like echography or to a surgical manipulation like in the context of surgical training. However, hydrogel materials fail to reproduce the complex internal state of strains and stresses that exist in biological tissues and result from the progressive tissue growth and remodeling under physiological loads. For instance, in the intervertebral disc, the different layers of the annulus fibrosus remodel and redistribute the intradiscal pressure applied by the nucleus [Michalek, A J et al. "Large residual strains are present in the intervertebral disc annulus fibrosus in the unloaded state" Journal of biomechanics vol. 45,7 (2012): 1227-31]. Similarly, the different layers composing the arterial walls remodel to distribute the stresses caused by blood pressure in such a way that the internal layer of endothelial cells is submitted to less tensile radial stresses than the outer muscular and collagen rich layers [Fung, Y C. "What are the residual stresses doing in our blood vessels?" Annals of biomedical engineering vol. 19,3 (1991): 237-49]. Such residual strains and stresses are also ubiquitous in plant walls and play a central role in their mechanical behavior and their morphogenesis.

It is also desirable that hydrogels be able to withstand a certain level of mechanical stresses without breaking. However, hydrogel materials generally have poor resistance to tensile loading, which greatly limits their use in implantology as well as in cell culture, in tissue engineering, in drug-release applications and in phantom applications where hydrogels can be subjected to tensile deformation due to physiological loadings or manipulation by an operator like a surgeon or by a machine like in a bioreactor.

Furthermore, it is highly desirable for an implant to be able to change size and/or geometry upon contact with a swelling agent, in particular water, to better fit a shape or put a structure under tension. Among other advantages, this makes it easier for the surgeon to implant it in the patient's body as he does not need to fix the implant while simultaneously applying tension like in the case of ligament and tendon reconstruction. This also helps the surgeon to put the implant under a state of tension that prevents the occurrence of buckling and reproduces better the physiological state of the native tissues.

Therefore, the invention aims at providing a material that can be used for manufacturing a human or animal implant or a tissue engineering scaffold or a tissue-mimicking phantom, said material being a hydrogel-based system mimicking the mechanical behavior of human or animal biological tissues and/or having an improved tensile stiffness in at least one direction and/or capable of self-adjusting.

### SUMMARY

The present invention relates to a dry fiber assembly comprising at least two fibers, the at least two fibers comprising at least one polymer consisting of at least two polymer chains, the at least two polymer chains being chemically or physically crosslinked with each other and oriented on a macromolecular scale in the dry state, the at least two fibers being non-woven, twisted, woven, braided or knitted together, or included within a polymeric matrix further comprised in the fiber assembly.

Advantageously, the at least two polymer chains are physically crosslinked with each other, the cross-linking corresponding to crystallites. More advantageously, the crystallinity of the polymer is greater than 15%, preferably it ranges from 20% to 80%, more preferably it ranges from 25% to 70%, even more preferably it ranges from 30% to 65%, the

crystallinity being measured by differential scanning calorimetry.

Advantageously, in each of the at least two fibers, the polymer chains are oriented on a macromolecular scale in the dry state in one direction, preferably in the direction of the fiber length.

Advantageously, the polymer chains are oriented on a macromolecular scale in the dry state in one direction after quenching below the glass transition temperature during the spinning process of the at least two fibers.

Advantageously, the linear density of each of the at least two fibers in the dry state is greater than $10^{-5}$ mg/m ($10^{-4}$ Dtex), preferably greater than $10^{-3}$ mg/m (0.01 Dtex), more preferably greater than 0.1 mg/m (1 Dtex), even more preferably greater than 1 mg/m (10 Dtex).

Advantageously, the polymer is selected from the group consisting of: poly(vinyl alcohol), poly(urethane)s and poly(ethylene glycol) and any copolymers thereof, preferably the polymer is poly(vinyl alcohol). More advantageously, the poly(vinyl alcohol) has a mass average molar mass $M_w$ ranging from 5,000 g/mol to 400,000 g/mol, preferably the poly(vinyl alcohol) has a mass average molar mass $M_w$ ranging from 25,000 g/mol to 300,000 g/mol, more preferably the poly(vinyl alcohol) has a mass average molar mass $M_w$ ranging from 80,000 g/mol to 200,000 g/mol.

In one embodiment, the at least two fibers are non-woven, twisted, woven, braided or knitted together, preferably the at least two fibers are twisted, woven, braided or knitted together, more preferably the at least two fibers are twisted or braided together, even more preferably the at least two fibers are twisted together.

In another embodiment, the at least two fibers are included within a polymeric matrix, said polymeric matrix being further comprised in the fiber assembly, wherein said polymeric matrix is made of a polymer selected from the group consisting of : poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof.

The present invention also relates to a swollen fiber assembly obtained by contacting the dry fiber assembly according to the invention with at least one swelling agent.

Advantageously, the weight of the at least one swelling agent represents at least 10% of the total weight of the swollen fiber assembly at room temperature, preferably the weight of the at least one swelling agent represents from 15% to 90% of the total weight of the swollen fiber assembly at room temperature, more preferably the weight of the at least one swelling agent represents from 20% to 80% of the total weight of the swollen fiber assembly at room temperature. Preferably, the room temperature is 20°C.

Advantageously, the elongation rate of the at least two fibers of the swollen fiber assembly is greater than 100%, preferably it is of at least 110%.

The present invention also relates to the use of the dry fiber assembly according to the invention for the reconstruction or replacement of a soft tissue in a mammal, in particular for the replacement or reinforcement of a soft tissue selected from the group consisting of: fatty tissues, muscles, tendons, ligaments, intervertebral disks, fasciae, skin, blood vessels, lymphatic vessels, cartilage, menisci and nerves.

The present invention also relates to the use of the swollen fiber assembly according to the invention for the reconstruction or replacement of a soft tissue in a mammal, in particular for the replacement or reinforcement of a soft tissue selected from the group consisting of: fatty tissues, muscles, tendons, ligaments, intervertebral disks, fasciae, skin, blood vessels, lymphatic vessels, cartilage, menisci and nerves.

The present invention also relates to a process for manufacturing a swollen fiber assembly according to the invention, comprising the following steps:

- providing at least two fibers in the dry state, said at least two fibers being made of a material comprising at least one polymer, the chains of the at least one polymer being chemically or physically crosslinked with each other and oriented on a macromolecular scale in the dry state,
- unweaving, twisting, weaving, braiding or knitting the at least two fibers together, or including them within a polymeric matrix, and
- contacting the at least two fibers, that are either non-woven, twisted, woven, braided or knitted together, or included within a polymeric matrix, with at least one swelling agent.

## DEFINITIONS

In the present invention, the following terms have the following meanings:

**"About",** before a figure or number, refers to plus or minus 10% of the face value of that figure or number. In one embodiment, "about", before a figure or number, refers to plus or minus 5% of the face value of that figure or number.

**"Axis of a fiber"** refers to the axis in the direction of the fiber length.

**"Axis of a yarn"** refers to the axis in the direction of the yarn length.

**"Axis of a structure"** refers to the axis in the direction of the longest dimension of a structure, in particular in the direction of the structure length. For example, the structure may be a fiber assembly having the form of a yarn, a twisted

fabric such as a cord, a woven fabric, a non-woven fabric, a braided fabric or a knitted fabric.

**"Comprising"** or **"comprise"** is to be construed in an open, inclusive sense, not limited to the features following this term.

**"Consisting of"** or **"consist"** is to be construed in a close, non-inclusive sense, limited to the features following this term.

**"Contraction rate"** refers to one minus the ratio between one dimension of a solid or structure measured in a given state and the same dimension in an initial state. Advantageously, the initial state may be a dry state. Advantageously, the given state may be a swollen state. Advantageously, the measured dimension is the length of the solid or structure, that is to say its longest dimension. In one embodiment, "contraction rate" refers to one minus the ratio between the length of a solid or structure measured in the swollen state and its length in the initial dry state; thus, in this case, the reference state for the contraction rate is the dry state. In one preferred embodiment, "contraction rate" refers to one minus the ratio between the length of a solid or structure measured in the swollen equilibrium state and its length in the initial dry state. Thus, the greater the contraction, the greater the contraction rate. A contraction rate of 0% corresponds to a solid or structure that has not contracted and a contraction rate of 50% corresponds to a solid or structure whose measured dimension, for example its length, has been divided by two.

**"Crosslink"** refers to a bond or a short sequence of bonds between two polymer chains. The crosslinks are chemical or physical. The chemical crosslinks correspond to covalent bonds between polymer chains. Preferably, the chemical crosslinks are obtained by using at least one of the following crosslinking methods: use of a multifunctional chemical agent (for example multi-aldehydes such as glutaraldehyde or glyoxal, multi-thiols, multi-acrylates or multi-carboxylic-acids, multi-dienes), use of irradiation (for example gamma-ray or electron-beam irradiation) or use of chemical reaction between reactive moieties of the polymer chains. The physical crosslinks correspond to noncovalent bonds between polymer chains (for example hydrogen bonds, crystallites, pi-pi stacking, ionic bonds, hydrophobic interactions). Preferably, the physical crosslinks are obtained by using at least one of the following crosslinking methods: solvent casting, freezing/thawing, liquid-liquid phase separation, heat treatment, crosslinking by ionic complexation (for example with borax); and crosslinking by mesophase separation in the case of copolymer chains.

**"Crystallite"** refers to a domain of a polymer having the same structure as a single crystal, that is a domain in which the atoms composing the polymer chains are arranged regularly onto a crystal lattice that extends in all directions. Within a crystallite, polymer chains are perfectly oriented parallel to each other along one crystalline axis. In one embodiment, the crystallites are insoluble in a swelling agent at the temperature of use. A **"semi-crystalline polymer"** refers to a polymer comprising crystallites and amorphous regions of said polymer, wherein the crystallites of said polymer are connected to each other by the amorphous regions of said polymer. The **"crystallinity"** of a semi-crystalline polymer refers to the fraction of polymer mass or the fraction of polymer segments that compose the crystallites over the total polymer mass or total number of polymer segments, respectively.

**"Dtex"** refers to the weight in grams of 10,000 meters of fiber.

**"Dry state"** refers to the state in the absence of swelling agent impregnation of a material, i.e. comprising less than 10% by weight of any swelling agent, preferably comprising less than 5% by weight of any swelling agent.

**"Elongation rate"** refers to the tensile deformation of a swollen fiber along the fiber axis. It is equal to the ratio of the length of the swollen fiber inside a structure over the length that the swollen fiber would have outside of said structure and free of external forces. The reference state for the elongation rate is the swollen state. An elongation rate of 100% corresponds to a fiber which length inside the structure is the same as the length it would have outside of the structure and free of external forces. An elongation rate of 200% corresponds to a fiber which length inside the structure is twice the length it would have outside of the structure and free of external forces. The elongation rate may be measured by using markers on the fibers, and measuring the length of the fibers in the free swollen state and in the swollen state inside a structure, in particular within a fiber assembly according to the invention.

**"Fiber"** refers to a thin, threadlike structure made of at least two polymer chains cross-linked with each other. According to the invention, a fiber in a dry state has a length ranging from 1 mm to 10 km, preferably ranging from 1 mm to 10 m, more preferably ranging from 1 mm to 1 m. According to the invention, a fiber in a dry state has a diameter ranging from 0.1 $\mu$m to 1000 $\mu$m, preferably ranging from 1 $\mu$m to 500 $\mu$m, more preferably ranging from 5 $\mu$m to 200 $\mu$m, even more preferably ranging from 10 $\mu$m to 150 $\mu$m.

**"From X to Y"** refers to the range of values between X and Y, the limits X and Y being included in said range.

**"Gel"** refers to a solid three-dimensional network of cross-linked polymer chains, percolating through a given volume. The gel can be swollen but not dissolved by a liquid (the liquid is referred to as **"swelling agent")** and exhibits the properties of a solid ranging from ductile to brittle. A chemical gel refers to a gel where the crosslinks are chemicals. A physical gel refers to a gel where the crosslinks are physicals. In a physical gel where crosslinks are crystallites, the crystallinity is typically greater than 5%, preferably the crystallinity ranges from 10% to 70%, more preferably from 20% to 60%, the crystallinity being measured by differential scanning calorimetry.

**"Hydrogel"** refers to a gel in which the swelling agent is water.

**"Hydrogel fibers"** refers to fibers made of a hydrogel, that is fibers made of a gel comprising a cross-linked

polymer matrix and water as the gel swelling agent.

**"Hydrolysis degree of PVA"** refers to the percentage of the number of vinyl alcohol monomers relative to the total number of monomers present in a PVA. Indeed, PVA is obtained from hydrolysis of poly(vinyl acetate) (PVAc) but this hydrolysis process is not perfect and there are always some remaining acetate functions along the chain; the hydrolysis degree of PVA gives the percentage of vinyl alcohol monomers.

**"Implant"** refers to an exogenous device inserted into the body of a mammal. **"Prosthesis"** or **"tissue substitutes"** refer to implants which are used to repair or replace an element of the body of a mammal, for example a soft biological tissue. In one embodiment, "implant" or "prosthesis" or "tissue substitutes" refers to an exogenous device inserted into the body of a human to repair or replace an element of said human body, for example a soft biological tissue such as a ligament, for example the anterior cruciate ligament (ACL) or an artery such as the aorta. In one embodiment, "implant" or "prosthesis" or "tissue substitutes" refers to an exogenous device inserted into the body of a human to repair or replace a biological tissue. In one embodiment, "implant" or "prosthesis" or "synthetic substitutes" refers to an exogenous device inserted into the body of a non-human mammal, such as horse or a dog, to repair or replace an element of said body, for example a soft biological tissue such as a ligament, for example the cranial cruciate ligament (CrCL).

**"Ligaments"** refers to a ligament of a mammal. In one embodiment, the ligament is selected from the group consisting of head and neck ligaments (cricothyroid ligament, periodontal ligament, suspensory ligament of the lens), wrist ligaments (palmar radiocarpal ligament, dorsal radiocarpal ligament, ulnar collateral ligament, radial collateral ligament), shoulder ligament (rotator cuff), and knee ligaments (anterior cruciate ligament (ACL), lateral collateral ligament (LCL), posterior cruciate ligament (PCL), medial collateral ligament (MCL), cranial cruciate ligament (CrCL) - quadruped equivalent of ACL, caudal cruciate ligament (CaCL) - quadruped equivalent of PCL, patellar ligament).

**"Oriented on a macromolecular scale in the dry state"** refers to a polymer material in which the segments of the backbone of polymer chains are, in the dry state, mostly oriented in one same direction within a macroscopic volume of said material. In the dry state of the material, the polymer chains do not have enough thermal energy or enough mobility to reorient in a more isotropic organization. When the polymer material oriented on a macromolecular scale in the dry state is put in contact with a swelling agent, the diffusion of the swelling agent molecules inside the material increases the mobility of the polymer chains and favors the elastic recoil of the oriented polymer segments into more random isotropic conformations. This recoil of oriented chain segments causes a contraction of the material along the direction of the main orientation of the segments of the backbone of polymer chains. A fiber according to the invention is oriented on a macromolecular scale in the dry state, i.e. the polymer chains composing the dry fiber have segments of their backbone mostly oriented along the fiber axis. In one embodiment, the term "mostly oriented in one same direction within a macroscopic volume of said material" means that the Hermans orientation factor is strictly greater than zero (Hermans orientation factor > 0). Advantageously, the Hermans orientation factor ranges from 0.1 to 1.0, more advantageously from 0.3 to 1.0.

In one embodiment, the dry state oriented on a macromolecular scale is obtained by stretching the polymer material in one direction above the glass transition temperature $T_g$ or the melting temperature $T_m$ of the oriented polymer segments and then cooling it below $T_g$ or $T_m$ respectively while maintaining the stretching.

In another embodiment, the segments of the backbone of polymer chains that are mostly oriented on a macromolecular scale in one same direction in the dry state have a glass transition temperature $T_g$ in the dry state which is greater than the temperature of assembly and/or storage, preferably greater than the room temperature. In this case, the main orientation of the segments of the backbone of polymer chains is released upon contact with a swelling agent which acts as a plasticizer.

In another embodiment, the polymer segments that are mostly oriented on a macromolecular scale in one same direction in the dry state assemble into crystalline domains for which the melting temperature $T_m$ in the dry state is greater than the temperature of assembly and/or storage, preferably greater than the room temperature; and the melting temperature $T_m$ in contact with the swelling agent is lower than the temperature of use, preferably lower than the temperature of mammals bodies. In this case, the main orientation of the polymer segments is released upon contact with a swelling agent which causes the melting of the oriented crystalline domains.

**"Pitch"** or **"lay length"** refers to the length of a yarn (or twisted structure made of yarns) on which a fiber (or yarn) makes a complete turn. It is equal to the inverse of the twisting rate.

**"Poly(urethane)s"** or **"PUs"** are a family of polymers with a backbone comprising -[NHC(=O)O]- groups. A polyurethane is typically produced by reacting an isocyanate with a polyol. PU chains contain at least two types of monomers alternating along the chain, one forming hard segments having reduced molecular flexibility and the other forming soft segments having an elastomeric behavior.

In one embodiment, PU is used as the at least one polymer of the at least two fibers according to the invention; in this embodiment, the monomers composing the soft segment either have a glass transition temperature in the dry state which is greater than 20°C or have a melting temperature in the dry state which is greater than 20°C and a melting temperature in the presence of a swelling agent which is lower than 20°C. The PU chains have a weight fraction of soft segments of at least 20 %, preferably more than 50 %, and the monomers composing the soft segments are preferentially selected from the

group consisting of: poly(ethylene oxide), poly(ethylene glycol), poly(hexamethylene oxide) and their copolymers.

**"Poly(vinyl alcohol)"** or **"PVA"** is a polymer with a hydrocarbon backbone and pendant hydroxyl groups, said polymer having the following structure:

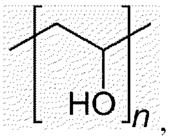,

wherein n is typically ranging from 100 to 10,000.

In one embodiment, PVA is used as the at least one polymer of the at least two fibers according to the invention; in this embodiment, the PVA chains have:

- a number of monomers ranging from 100 to 10,000, preferably from 500 to 7,000;
- a mass average molar mass ($M_w$) ranging from 5,000 to 400,000 g/mol, preferably from 25,000 to 300,000 g/mol, even more preferably from 80,000 to 200,000 g/mol;
- a glass transition temperature ($T_g$) ranging from 50°C to 95°C, preferably from 60°C to 89°C;
- a melting point ranging from 200°C to 270°C, preferably from 210°C to 250°C;
- and/or a hydrolysis degree ranging from 70% to 100%, preferably from 80% to 99.9%.

In one embodiment, PVA is used as a polymeric matrix entrapping the at least two fibers and the at least one swelling agent according to the invention; in this embodiment, the PVA chains have:

- a number of monomers ranging from 100 to 10,000, preferably from 500 to 7,000;
- a mass average molar mass ($M_w$) ranging from 5,000 to 400,000 g/mol, preferably from 25,000 to 300,000 g/mol, even more preferably from 80,000 to 200,000 g/mol;
- a glass transition temperature ($T_g$) ranging from 50°C to 95°C, preferably from 60°C to 89°C;
- a melting point ranging from 200°C to 270°C, preferably from 210°C to 250°C;
- and/or a hydrolysis degree ranging from 70% to 100%, preferably from 80% to 99.9%. In one embodiment, PVA is used as a coating of the fiber assembly according to the invention; in this embodiment, the PVA chains have:
- a number of monomers ranging from 100 to 10,000, preferably from 500 to 7,000;
- a mass average molar mass ($M_w$) ranging from 5,000 to 400,000 g/mol, preferably from 25,000 to 300,000 g/mol, even more preferably from 80,000 to 200,000 g/mol;
- a glass transition temperature ($T_g$) ranging from 50°C to 95°C, preferably from 60°C to 89°C;
- a melting point ranging from 200°C to 270°C, preferably from 210°C to 250°C;
- and/or a hydrolysis degree ranging from 70% to 100%, preferably from 80% to 99.9%.

**"Polymer"** refers to a material comprising at least two polymer chains.

**"Polymer chain"** refers to a linear or branched polymer chain, the backbone of which typically comprises more than 10 monomers linked covalently in series, preferably more than 100 monomers.

**"Polymer segment"** means a portion of at least 2 consecutive covalent bonds of the backbone of a chain of that polymer.

**"Residual strains"** and **"residual stresses"** are the strains and stresses that remain in a solid or structure when the external loads are removed.

**"Room temperature"** refers to a temperature ranging from 20°C to 25°C. Unless specified otherwise, a specific "room temperature" refers to a temperature of 20°C.

**"Self-adjusting system"** refers to a system that is able to change size and/or geometry (shape) without a mechanical action from the user to better fit a shape or put a structure under tension. Advantageously, the change in size and/or geometry is produced by the contact with a swelling agent, preferably with an aqueous liquid, more preferably with water.

**"Soft biological tissues"** or **"soft tissues"** refers to the non-mineralized tissues of a mammal, for example a tissue selected from the group consisting of: fatty tissues, muscles, tendons, ligaments, fasciae, skin, blood vessels, lymphatic vessels, cartilage, menisci and nerves. In an embodiment, "soft biological tissues" or "soft tissues" refers to tendons and ligaments. In a preferred embodiment, "soft biological tissues" or "soft tissues" refers to a ligament. In a more preferred embodiment, "soft biological tissues" or "soft tissues" refers to anterior cruciate ligament (ACL).

**"Swelling agent"** refers to a fluid, in particular a liquid, used to swell a gel, network or solid. In one embodiment, "swelling agent" refers to a liquid, used to swell a gel, network or solid, said liquid being selected from the group consisting

of: water, aqueous solutions and biological fluids.

**"Twisting rate"** or **"twist"** refers to the number of turns per unit length of fiber (in a yarn) or of yarn (in a twisted structure made of yarns). It is equal to the inverse of the pitch.

**"Yarn"** refers to an example of fiber assembly according to the invention, that comprises at least two fibers being made of a material comprising at least one polymer, the chains of the at least one polymer being chemically or physically crosslinked with each other and oriented on a macromolecular scale in the dry state, the at least two fibers being twisted together, the twisting of the at least two fibers being carried out with the at least two fibers in the dry state.

**"Yarn-structure angle $\alpha$"** refers to the angle formed between the axis of a yarn and the axis of the structure.

**"Fiber-yarn angle $\beta$"** refers to the angle formed between the axis of a fiber and the axis of the yarn.

## DETAILED DESCRIPTION

### Dry fiber assembly

This invention relates to a dry fiber assembly comprising at least two fibers, the at least two fibers comprising at least one polymer consisting of at least two polymer chains, the at least two polymer chains being chemically or physically crosslinked with each other and oriented on a macromolecular scale in the dry state, the at least two fibers being either non-woven, twisted together, woven together, braided together, knitted together, or included within a polymeric matrix further comprised in the dry fiber assembly.

The assembly of the at least two fibers with each other or in the matrix is carried out with the at least two fibers in the dry state.

#### Crosslinking

The polymer chains of the fibers are chemically and/or physically crosslinked with each other.

The crosslinking between the polymer chains of the fibers signifies that there is at least one crosslink between one polymer chain and at least one other polymer chain. The number of cross-links must be sufficient to obtain a gel.

In one embodiment, the polymer chains of the fibers are chemically crosslinked with each other. Preferably, the polymer chains of the fibers are chemically crosslinked with each other by using at least one of the following crosslinking methods: use of chemical agents (for example glutaraldehyde, glyoxal, maleic acid, citric acid, trisodium trimetaphosphate, sodium hexametaphosphate, dianhydrides, succinic acid, and sulfosuccinic acid) and use of irradiation (for example gamma-ray or electron-beam irradiation). In another embodiment, the polymer chains of the fibers are physically crosslinked with each other. Preferably, the polymer chains of the fibers are physically crosslinked with each other using at least one of the following crosslinking methods: crosslinking by crystallites in the case of polymer chains with crystallizable segments using solvent casting, freezing/thawing, liquid-liquid phase separation and heat treatment; crosslinking by ionic complexation (for example with borax); and crosslinking by mesophase separation in the case of copolymer chains.

In another embodiment, the polymer chains of the fibers are chemically and physically crosslinked with each other. Preferably, the polymer chains of the fibers are chemically crosslinked with each other by using at least one of the following crosslinking methods: use of chemical agents (for example glutaraldehyde, glyoxal, maleic acid, citric acid, trisodium trimetaphosphate, sodium hexametaphosphate, dianhydrides, succinic acid, and sulfosuccinic acid) and use of irradiation (for example gamma-ray or electron-beam irradiation); and physically crosslinked with each other by using at least one of the following crosslinking methods: crosslinking by crystallites in the case of polymer chains with crystallizable segments using solvent casting, freezing/thawing, liquid-liquid phase separation and heat treatment; crosslinking by ionic complexation (for example with borax); and crosslinking by mesophase separation in the case of copolymer chains.

In one embodiment, the manufacture of the fibers according to the invention comprises a step of physically and/or chemically crosslinking the polymer chains of the fibers. The person skilled in the art knows how to adapt the crosslinking method and/or the crosslinking conditions depending for instance on the type of the polymer(s) comprised in the fibers and on the desired crosslinking rate.

Advantageously, the crosslinking between the polymer chains of the fibers corresponds to crystallites. More advantageously, the crystallinity of the fibers, measured by wide angle X-ray diffraction, is greater than 10%, preferably from 15% to 70%, more preferably from 20% to 60%, even more preferably from 25% to 55%.

Advantageously, the crosslinking between the polymer chains of the fibers corresponds to crystallites. More advantageously, the crystallinity of the fibers, measured by differential scanning calorimetry, is greater than 15%, preferably it ranges from 20% to 80%, more preferably it ranges from 25% to 70%, even more preferably it ranges from 30% to 65%.

Orientation of the polymer chains

The chains of the at least one polymer in a fiber are oriented on a macromolecular scale in said fiber in the dry state, preferably they are oriented in the direction of the fiber length.

Advantageously, the preferential orientation of the chains of the at least one polymer in one particular direction in the fiber is characterized by birefringence measurement [Kolbuk et al. "Optical birefringence and molecular orientation of electrospun polycaprolactone fibers by polarizing-interference microscopy" European Polymer Journal vol. 48 (2012): 275-83], X-ray fiber diffraction [Lafrance, C P et al. "Study of the distribution of molecular orientation in highly oriented polyethylene by x-ray diffraction" Macromolecules vol. 24 (1991): 4948-56], acoustic velocity [Moseley W W "The measurement of molecular orientation in fibers by acoustic methods" Journal of applied polymer science vol. 3 (1960): 266-76], polarized infrared (IR) spectroscopy [Boulet-Audet M et al. "Attenuated total reflection infrared spectroscopy: an efficient technique to quantitatively determine the orientation and conformation of proteins in single silk fibers" Applied spectroscopy vol. 62 (2008): 956-62], and polarized Raman spectroscopy [Rousseau M-E et al. "Study of protein conformation and orientation in silkworm and spider silk fibers using Raman microspectroscopy" Biomacromolecules vol. 5 (2004):2247-57].

More advantageously, the preferential orientation of the chains of the at least one polymer in one particular direction in the fiber is determined by wide-angle x-ray scattering measurements. It can be quantified by measuring the Hermans orientation factor [Lafrance, C P et al. "Study of the distribution of molecular orientation in highly oriented polyethylene by x-ray diffraction" Macromolecules vol. 24 (1991): 4948-56]. The Hermans orientation factor is a scalar descriptor, which quantifies the orientation of the normal to a diffraction plane with respect to a reference direction. If the fiber axis is chosen as the reference direction, this factor is null when the distribution is isotropic and ranges from -0.5 for a perfect orientation of the chains perpendicular to the fiber axis, to 1 for a perfect orientation of the chains parallel to the fiber axis.

In one embodiment, the chains of the at least one polymer are oriented in the fiber on a macromolecular scale in the dry state with a preferential orientation in one direction, preferably said direction is the direction of the fiber length. Preferably, the orientation of the chains of the at least one polymer in one direction on a macromolecular scale in the dry state is obtained by stretching the at least two fibers in the dry state above the glass transition temperature or the melting temperature of some of the polymer segments and then cooling it below this glass transition temperature or melting temperature while maintaining the stretching. Alternatively, the orientation of the chains of the at least one polymer in one direction on a macromolecular scale is obtained by stretching the at least two fibers in a swollen state and then drying it while maintaining the stretching. Alternatively, the orientation of the chains of the at least one polymer in one direction on a macromolecular scale is obtained by drying or by cooling below the glass transition temperature or melting temperature of some of the polymer segments during the spinning process of the at least two fibers.

Linear density

In one embodiment, the linear density of each of the at least two fibers in the dry state is greater than $10^{-5}$ mg/m (corresponding to $10^{-4}$ Dtex), preferably greater than $10^{-3}$ mg/m (corresponding to 0.01 Dtex), more preferably greater than 0.1 mg/m (corresponding to 1 Dtex), even more preferably greater than 1 mg/m (corresponding to 10 Dtex).

The polymer

The polymer of the at least two fibers of the dry fiber assembly according to the invention comprises at least one polymer whose backbone contains at least one polymer segment. In one embodiment, the at least one polymer segment has a glass-transition temperature $T_g$ in its dry state which is at least 20°C greater than the temperature of use. Preferably, the temperature of use is room temperature (20°C-25°C, preferably 20°C) or body temperature (37°C). In another embodiment, the at least one segment has a melting temperature $T_m$ in the dry state which is at least 5°C greater than the temperature of use, and a melting temperature in contact with a swelling agent which is at least 5°C lower than the temperature of use. Preferably, the temperature of use is the room temperature (20°C-25°C, preferably 20°C) or body temperature (37°C).

In one embodiment, the polymer is selected from the group consisting of: poly(vinyl alcohol), poly(ethylene glycol), poly(urethane)s, poly(acrylamide)s, poly(N,N-dimethylacrylamide), poly(acrylic acid), poly(acrylate)s, poly(2-hydroxyethyl methacrylate), poly(saccharide)s, proteins, synthetic polypeptides, and any copolymers thereof. In this embodiment, the at least one swelling agent is preferably selected from the group consisting of: water and polar solvents.

In one embodiment, the polymer is selected from the group consisting of: poly(amide)s, aromatic poly(ester)s (in particular poly(ethylene terephthalate)), poly(vinyl chloride), poly(tetra fluoroethylene), aliphatic poly(ester)s, and any copolymers thereof. In this embodiment, the at least one swelling agent is preferably selected from the group consisting of: apolar or slightly polar solvents.

In one embodiment, the polymer comprises poly(vinyl alcohol). Preferably, the polymer is poly(vinyl alcohol) (PVA).

Advantageously, the PVA is highly hydrolyzed, in particular the hydrolysis degree of PVA is equal or greater than 90%, more preferably the hydrolysis degree of PVA is greater than 95%, even more preferably the hydrolysis degree of PVA is from 98% to 99.99%. A highly hydrolyzed PVA, in particular a PVA having a hydrolysis degree equal or greater than 90%, advantageously greater than 95%, more advantageously from 98% to 99.99%, is preferred because acetate groups have been reported to be pro-inflammatory; in addition, they decrease the crystallization of the PVA and increase the solubility of the PVA.

Advantageously, the PVA has a melting point of about 230°C.

Advantageously, the glass transition temperature ($T_g$) of the PVA in its dry state is from 70°C to 90°C, preferably the glass transition temperature ($T_g$) of the PVA is 85°C.

More advantageously, the PVA has a hydrolysis degree from 98% to 99.99%, a melting point of 230°C and a glass transition temperature ($T_g$) of 85°C.

Preferably, the PVA has an average molar mass ranging from 25,000 g/mol to 300,000 g/mol. More preferably, the PVA has an average molar mass ranging from 80,000 g/mol to 200,000 g/mol.

PVA hydrogels have the advantages of being materials that are stable, non-resorbable and very well tolerated *in vivo*: they have good biocompatibility characteristics, provided that the hydrolysis degree is equal to or greater than 90%.

In one embodiment, the at least two fibers are made of a different polymer from each other. For example, among the at least two fibers, at least one fiber may be made of PVA and at least one fiber may be made of a polymer selected from the group consisting of: poly(ethylene glycol), poly(urethane)s, poly(acrylamide)s, poly(N,N-dimethylacrylamide)), poly(acrylic acid), poly(acrylate)s, poly(2-hydroxyethyl methacrylate), poly(amide)s, aromatic poly(ester)s (in particular poly(ethylene terephthalate)), poly(vinyl chloride), poly(tetra fluoroethylene), aliphatic poly(ester)s, poly(saccharide)s, proteins, synthetic polypeptides, and any copolymers thereof.

In another embodiment, the at least two fibers are made of the same material. For example, both fibers may be made of PVA.

In one embodiment, in at least one fiber of the at least two fibers, the polymer chains may be made of different polymer from each other. For example, at least one fiber of the at least two fibers may comprise at least one PVA chain and at least one poly(urethane) or poly(ethylene glycol) chain. In one embodiment, the at least two fibers each comprise at least one PVA chain and at least one poly(urethane) or poly(ethylene glycol) chain.

Form of the dry fiber assembly

The at least two fibers of the dry fiber assembly according to the invention are either non-woven, twisted, woven, braided or knitted together, or included within a polymeric matrix further comprised in the fiber assembly.

In one embodiment, the at least two fibers of the dry fiber assembly according to the invention are at least one of: twisted, non-woven, woven, braided, knitted together and any combinations thereof. In one preferred embodiment, the at least two fibers of the dry fiber assembly according to the invention are twisted. In another preferred embodiment, the at least two fibers of the dry fiber assembly according to the invention are braided and woven.

The twisting, non-weaving, weaving, braiding, knitting or inclusion within a polymeric matrix may concern part or all the length of the at least two fibers of the dry fiber assembly according to the invention.

Advantageously, the dry fiber assembly according to the invention has a shape selected from the group consisting of: a twisted fabric such as a rope, a non-woven fabric, a woven fabric, a braided fabric and a knitted fabric. Advantageously, the dry fiber assembly according to the invention has a shape of a twisted fabric such as a rope. More advantageously, the dry fiber assembly according to the invention has a shape of a twisted double helix rope structure. Even more advantageously, the dry fiber assembly according to the invention has a shape of a twisted double helix rope structure in which the ratio l/L between the fiber length 1 and the dry fiber assembly length L in the same direction is equal to or greater than 1.2.

The inventors have surprisingly discovered that a dry fiber assembly according to the invention is able to mimic the residual stresses of human or animal biological tissues and/or have an improved tensile stiffness in at least one direction and/or be capable of self-adjusting. In particular, the friction forces between the at least two fibers that are non-woven, twisted, woven, braided or knitted together, maintain the fiber assembly in a constrained state when the assembly is contacted with a swelling agent, thereby putting the fibers of the fiber assembly into tension when the fiber assembly is contacted with the swelling agent; this allows the fiber assembly according to the invention to have a high stiffness, non-zero residual stresses and the ability to self-adjust during the swelling.

Advantageously, the dry fiber assembly according to the invention comprises 8 to 24 strands, preferably 12 to 16 strands, composed of 6 to 24 yarns, preferably 10 to 20 yarns, organized into bundles of 1 to 6 strands, preferably 2 to 4 strands, and twisted together with a twisting rate from 0.1 tr/cm to 10 tr/cm, preferably with a twisting rate from 0.2 tr/cm to 5 tr/cm, more preferably with a twisting rate from 0.2 tr/cm to 2 tr/cm, wherein each yarn comprises 10 to 1000 fibers,

preferably 10 to 100 fibers, more preferably 15 fibers, twisted together before assembly with a twisting rate from 0.1 to 2 tr/cm, preferably with a twisting rate of 0.4 to 1.5 tr/cm, more preferably with a twisting rate of 1.25 tr/cm.

The inventors have surprisingly discovered an original structural effect exploiting the confined swelling within the structures to put the fibers in an elongated state. This effect allows modifying the mechanical behavior of the swollen hydrogel fiber assemblies and in particular to obtain twisted structures which stiffness increases with the twisting rate, which is not achievable with twisted assemblies made of dry fibers or made of fibers that were swollen before assembly (see Example 3). Therefore, the dry fiber assemblies according to the invention are easier to handle and have a higher stiffness than the same fiber assembly made of fibers that were swollen before assembly.

In another embodiment, the at least two fibers of the dry fiber assembly according to the invention are included within a polymeric matrix further comprised in the dry fiber assembly, wherein said polymeric matrix is not soluble in a swelling agent. In this case, the contractile internal stresses created by the fiber assembly can put this matrix under compression, therefore increasing its resistance to tension, in a similar manner to pre-stressed concrete.

Advantageously, the dry fiber assembly according to the invention has the shape of a tube, wherein the at least two fibers may have different orientations from each other with respect to the tube axis (which corresponds to the length direction of said tube).

Advantageously, the dry fiber assembly according to the invention has the shape of a sheet, wherein the at least two fibers may have different orientations from each other within the plane of the sheet.

Advantageously, the dry fiber assembly according to the invention has the shape of a block, wherein the block may have any geometry, such as a cube, a cuboid, a parallelepiped, a sphere, a disc, a cylinder, a cone or a pyramid.

In still another embodiment, the at least two fibers of the dry fiber assembly according to the invention are non-woven, twisted, woven, braided or knitted together, wherein the resulting twisted fabric such as a non-woven fabric, a rope, a woven fabric, a braided fabric or a knitted fabric is included within a polymeric matrix further comprised in the dry fiber assembly.

Polymeric matrix

In one embodiment, the dry fiber assembly according to the invention comprises at least two fibers and a polymeric matrix entrapping the at least two fibers. The at least two fibers may be either entrapped individually in the polymeric matrix, or entrapped in the form of a yarn of at least two fibers in the polymeric matrix, or entrapped in the form of a twisted fabric such as a rope, a non-woven fabric, a woven fabric, a braided fabric or a knitted fabric in the polymeric matrix.

Advantageously, the polymeric matrix is made of a polymer selected from the group consisting of: poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof.

The presence of the polymeric matrix replaces the interactions between the at least two fibers that are non-woven, twisted, woven, braided or knitted together, maintaining the fiber assembly in a constrained state when the assembly is contacted with a swelling agent, thereby putting the fibers of the fiber assembly into tension when said fiber assembly is contacted with the swelling agent; this allows the fiber assembly according to the invention to have a higher stiffness than the same assembly with non-elongated fibers, non-zero residual stresses and the ability to self-adjust during the swelling.

Dry state of the at least two fibers

In the dry fiber assembly according to the invention, the assembly of the at least two fibers with each other and/or within a polymeric matrix is carried out with the at least two fibers in the dry state.

Thus, advantageously, the process for manufacturing a dry fiber assembly according to the invention comprises a step of assembling the at least two fibers with each other and/or in a polymeric matrix, said step being carried out with the at least two fibers in the dry state. The present invention also relates to a dry fiber assembly obtained by the process according to the invention for manufacturing a dry fiber assembly according to the invention.

Indeed, the inventors have surprisingly discovered that the contraction of the at least two fibers induced by the contact with a swelling agent is constrained within a fiber assembly according to the invention and puts the fibers under tension. This allows the fiber assembly according to the invention to have a higher stiffness after swelling than the same fiber assembly made of fibers swollen before assembly. This also allows the fiber assembly according to the invention to have non-zero residual stresses, whose intensity and direction can be adjusted, mimicking for instance the residual stresses in biological tissues. In addition, the fiber assembly according to the invention is able to self-adjust during the swelling.

Coating of the dry fiber assembly according to the invention

According to an embodiment, the dry fiber assembly according to the invention is coated with a polymer layer. Preferably, the polymer of said polymer layer is selected from the group consisting of poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof. More preferably, said polymer layer is a poly(vinyl alcohol) layer.

Advantageously, the polymer layer of the coating may also be a composite of a polymer and inorganic fillers, in particular fillers composed of hydroxyapatite or calcium phosphate. This allows to improve the bone integration of the implants.

According to a preferred embodiment, the dry fiber assembly according to the invention has a shape selected from the group consisting of: a non-woven fabric, a twisted fabric such as a cord, a woven fabric, a braided fabric and a knitted fabric, and is coated with a polymer selected from the group consisting of poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and their copolymers; preferably, the dry fiber assembly according to the invention is coated with poly(vinyl alcohol).

The coating of the dry fiber assembly, in particular the poly(vinyl alcohol) coating, allows an easier insertion of the dry fiber assembly in the body of the patient in need, and therefore it facilitates the gesture of the surgeon.

In one embodiment, the coating of the swollen fiber assembly, in particular the poly(vinyl alcohol) coating, allows to reduce the friction against other surfaces and allows an easier insertion of the swollen fiber assembly in the body of the patient in need, and therefore it facilitates the gesture of the surgeon.

In one embodiment, the coating of the swollen fiber assembly, in particular the composite coating, allows to incorporate bioactive substances that favor the integration in the body and reduce the fibrous encapsulation induced by the inflammatory response.

Advantages of the dry fiber assembly according to the invention

The inventors have surprisingly found that a dry fiber assembly according to the invention is self-adjusting and has an improved tensile stiffness in at least one direction. Thus, it can be used as a material for manufacturing a mammal prosthesis or a device used inside the body of a mammal. It can also be used as a mammal prosthesis or as a device used inside the body of a mammal.

The inventors have surprisingly found that the dry fiber assembly according to the invention when put in contact with a swelling agent have residual stresses whose intensity and direction are adjustable. In particular, the contraction of the fibers within the fiber assembly can put parts of the structure under compressive internal stresses. The presence of such compressive internal stresses provides a way to reduce the risk of damage when the material is put under tensile loading and therefore it can enhance the durability of the material. Thus, it can be used as a material for manufacturing a mammal prosthesis or a device used inside the body of a mammal. It can also be used as a mammal prosthesis or as a device used inside the body of a mammal.

The inventors have surprisingly found that the residual stresses induced by the contact of the dry fiber assembly with a swelling agent can mimic the residual stresses and strains in biological tissues. The dry fiber assembly according to the invention can be used as a material for manufacturing a synthetic device which reproduces the way stresses are distributed in biological tissues under physiological loadings. For instance, such biomimetic residual stresses allow to implant devices in the body of a mammal with less perturbation of the mechanical stresses in the surrounding tissues, therefore it reduces the risk of unwanted side effects like an alteration of the blood flow. Thus, it can be used as a material for manufacturing a mammal prosthesis or a device used inside the body of a mammal. It can also be used as a mammal prosthesis or as a device used inside the body of a mammal. It can also be used as a material for manufacturing a tissue-mimicking phantom for the calibration of bioimaging techniques or for surgical training or for surgical planning. It can also be used as a material for manufacturing a substrate, matrix or scaffold for cell culture, in particular for the case of *in vitro* cell culture in bioreactors.

**Swollen fiber assembly**

The present invention also relates to a swollen fiber assembly obtained by contacting the dry fiber assembly according to the invention as described above with at least one swelling agent.

Advantageously, all the features and advantageous features described in part "Dry fiber assembly" of the present application apply *mutatis mutandis* for the swollen fiber assembly according to the invention.

Swelling agent

The swollen fiber assembly according to the invention comprises at least one swelling agent. The at least one swelling agent is suitable for swelling the at least two fibers of the dry fiber assembly according to the invention as described above.

Advantageously, the at least one swelling agent is selected from the group consisting of: water, dimethyl sulfoxide (DMSO), dimethylformamide (DMF), chloroform, trichloroethylene, orthodichlorobenzene and orthoxylene, toluene, trichloroethylene, n-butyl acetate, tetrahydrofuran, methylene chloride, methyl alcohol, ethyl alcohol, benzyl alcohol, ethylene glycol and mixtures thereof.

More advantageously, the at least one swelling agent is selected from the group consisting of: water, dimethyl sulfoxide (DMSO), dimethylformamide (DMF) and mixtures thereof.

If the at least two fibers consist of a material comprising or consisting of poly(vinyl alcohol), poly(ethylene glycol), poly(urethane)s, poly(acrylamide)s, poly(N,N-dimethylacrylamide), poly(acrylic acid), poly(acrylate)s, poly(2-hydroxyethyl methacrylate), poly(saccharide)s, proteins, or synthetic polypeptides, then the at least one swelling agent is preferably selected from the group consisting of: water, dimethyl sulfoxide (DMSO), dimethylformamide (DMF) and mixtures thereof.

If the at least two fibers consist of a material comprising or consisting of a non-hydrophilic polymer such as poly(ethylene terephthalate), then the at least one swelling agent is preferably selected from the group consisting of: dimethyl sulfoxide (DMSO), chloroform, trichloroethylene, orthodichlorobenzene, orthoxylene, toluene, benzoic acid and mixtures thereof.

If the at least two fibers consist of a material comprising or consisting of a non-hydrophilic polymer such as poly(vinyl chloride), then the at least one swelling agent is preferably selected from the group consisting of: trichloroethylene, toluene, n-butyl acetate, and mixtures thereof.

If the at least two fibers consist of a material comprising or consisting of a non-hydrophilic fluoropolymer such as poly(tetra fluoroethylene), then the at least one swelling agent is preferably selected from the group consisting of: dimethyl sulfoxide (DMSO), tetrahydrofuran, methylene chloride and mixtures thereof.

If the at least two fibers consist of a material comprising or consisting of polyamide, then the at least one swelling agent is preferably selected from the group consisting of: methyl alcohol, ethyl alcohol, and benzyl alcohol, water, and ethylene glycol and mixtures thereof.

When the swelling agent is water, the swollen fiber assembly according to the invention is a hydrogel fiber assembly.

In one embodiment, the weight of the at least one swelling agent represents at least 10% of the total weight of the swollen fiber assembly at room temperature, preferably the weight of the at least one swelling agent represents from 15% to 90% of the total weight of the swollen fiber assembly at room temperature, more preferably the weight of the at least one swelling agent represents from 20% to 80% of the total weight of the swollen fiber assembly at room temperature. Preferably, the room temperature is 20°C.

Advantages of the swollen fiber assembly according to the invention

The crosslinking of the polymer chains of the at least two fibers of the dry fiber assembly according to the present invention allows said fibers to be insoluble in a swelling agent, in particular in water and aqueous solutions. When the crosslinked polymer network is immerged into a swelling agent, it swells and forms a solid gel, composed of the swelling agent osmotically trapped in the swollen polymer network. In particular, when the crosslinked PVA network is immerged into water, it swells and forms a solid called "hydrogel", composed of water osmotically trapped in the swollen PVA network.

This phenomenon is illustrated in **Figure 15.** In **Figure 15A,** it can be seen a dry fiber according to the invention (for example a dry PVA fiber) with length $l_d$, that contracts longitudinally and increases in diameter upon swelling in a swelling agent (for example water) to form a swollen fiber (for example a PVA hydrogel fiber) with equilibrium length $l_{eq}$. In **Figure 15B,** it can be seen dry fibers that are assembled inside a textile or composite; upon swelling (in particular in water), fiber-fiber or fiber-matrix friction impedes the full contraction of the fibers which remain in a stretched state with a length $l_s$ greater than the equilibrium length $l_{eq}$.

In the swollen fiber assembly according to the invention, the length of the at least two fibers are greater than the length of the same fibers alone and at the swelling equilibrium: $l_s > l_{eq}$. Otherwise expressed, the elongation rate of the at least two fibers of the swollen fiber assembly according to the invention is greater than 100%, preferably it is of at least 110%.

In the swollen fiber assembly according to the invention, the at least two fibers are stretched which allows to increase the rigidity of the assembly as compared to another swollen assembly having the same structure but obtained from an assembly of pre-swollen fibers.

When the fiber assembly according to the invention further comprises a polymeric matrix entrapping the at least

two fibers or a coating around said fiber assembly, said polymeric matrix or coating being poorly resistant to tension, as it is often the case for brittle hydrogels, then the contractile internal stresses created by the fiber assembly can put this polymeric matrix or coating under compression, therefore increasing its resistance to tension, in a similar manner to pre-stressed concrete.

**Use of the dry and swollen fiber assemblies**

The invention also relates to the use of a fiber assembly according to the invention (the generic term "a fiber assembly according to the invention" refers to the dry fiber assembly according to the invention as described above and/or the swollen fiber assembly according to the invention as described above), for the reconstruction or replacement of soft tissues in a mammal. Preferably, the mammal is selected from the group consisting of a human, a horse and a dog. More preferably, the mammal is a human. More preferably, the mammal is a dog.

In one embodiment, the invention relates to the use of a fiber assembly according to the invention, for the reconstruction or replacement of any ligament in a mammal. Ligaments which can be repaired may be selected from the group consisting of: head and neck ligaments (cricothyroid ligament, periodontal ligament, suspensory ligament of the lens), wrist ligaments (palmar radiocarpal ligament, dorsal radiocarpal ligament, ulnar collateral ligament, radial collateral ligament), shoulder ligament (rotator cuff), knee ligament (anterior cruciate ligament (ACL), lateral collateral ligament (LCL), posterior cruciate ligament (PCL), medial collateral ligament (MCL), cranial cruciate ligament (CrCL) - quadruped equivalent of ACL, caudal cruciate ligament (CaCL) - quadruped equivalent of PCL, and patellar ligament.

In one embodiment, the invention relates to the use of a fiber assembly according to the invention, for the reconstruction or replacement of any tendon in a mammal. Tendons which may be repaired may be selected from the group consisting of: shoulder tendons (teres minor tendons, infraspinatus tendons, supraspinatus tendons, subscapularis tendons), elbow and forearm tendons (deltoid tendons, biceps tendons, triceps tendons, brachioradialis tendons, supinator tendons), wrist tendons (flexor carpi radialis tendons, flexor carpi ulnaris tendons, extensor carpi radialis tendons, extensor carpi radialis brevis tendons), hip and leg tendons (iliopsoas tendons, obturator internus tendons, adductor longus, brevis and magnus tendons, gluteus maximus and gluteus medius tendons), knee tendons (quadriceps tendons including the patellar tendon, hamstring tendons, sartorius tendons), ankle tendons (gastrocnemius tendons including the Achilles tendon, soleus tendons, tibialis anterior tendons, peroneus longus tendons), hands and feet tendons (flexor digitorum longus tendons, interosseus tendons, flexor digitorum profundus tendons, abductor digiti minimi tendons, opponens pollicis tendons, flexor pollicis longus tendons, extensor and abductor pollicis tendons, flexor hallucis longus tendons, flexor digitorum brevis tendons, lumbrical tendons, abductor hallucis tendons, flexor digitorum longus tendons, abductor digiti minimi tendons), head and neck tendons (ocular tendons, levator palpebrae tendons, masseter tendons, temporalis tendons, trapezius tendons, sternocleidomastoid tendons, semispinalis capitis and splenius capitis tendons, mylohyoid and thyrohyoid tendons, sternohyoid tendons), and torso tendons (rectus abdominis tendons, external oblique tendons, transversus abdominis tendons, latissimus dorsi tendons, erector spinae tendons).

In one embodiment, the invention relates to the use of a fiber assembly according to the invention, for the reconstruction or replacement of other soft osteoarticular tissues of interest, such as the tissues selected from the group consisting of: fascia, intervertebral disk (complete or annulus fibrosus only), menisci and cartilage.

In one embodiment, the invention relates to the use of a fiber assembly according to the invention, for the reconstruction or replacement of cardiovascular tissues in a mammal. Cardiovascular tissues which can be repaired may be selected from the group consisting of: aorta, hepatic arteries, coronary arteries, peripheral veins and arteries, vein valves and heart valves.

In one embodiment, the invention relates to the use of a fiber assembly according to the invention as a material for manufacturing a prosthesis or a tissue engineering scaffold, in particular a soft tissue replacement material.

In one embodiment, the invention relates to the use of a fiber assembly according to the invention as a material for manufacturing an implantable device, in particular a surgical mesh implant. The surgical textiles and meshes may for example be selected from the group consisting of: hernia mesh and transvaginal mesh implants.

In one embodiment, the invention relates to the use of a fiber assembly according to the invention to form or replace a joint in a medical device. Preferably, said medical device is selected from the group consisting of a robotic structure, an orthosis, a prosthesis and an exoskeleton.

In one embodiment, the invention relates to the use of a fiber assembly according to the invention to form a substrate or matrix for *in vitro* cell culture, in particular for the case of cell culture in bioreactors allowing the application of mechanical stresses.

In one embodiment, the invention relates to the use of a fiber assembly according to the invention as a tissue-mimicking phantom. A tissue-mimicking phantom is used for the development and calibration of imaging techniques, medical treatments and protocols, biomedical research, training and surgical planning. In a preferred embodiment, the invention relates to the use of a fiber assembly according to the invention to form a phantom tissue for imaging.

In one embodiment, the invention relates to the use of a fiber assembly according to the invention to form model

tissues for surgical planning, surgical training or pedagogical activities.

In one embodiment, the invention relates to the use of a fiber assembly according to the invention for at least one use selected from the group consisting of: implantable tissue substitute, drug delivery implant, cell therapy vehicle and matrices, phantom tissue for imaging, biomimetic tissue, and culture matrix substitutes for research and teaching.

Advantageously, the uses of a fiber assembly according to the invention are *ex vivo* and/or *in vivo.* More advantageously, the uses of a fiber assembly according to the invention are *in vivo.* Alternatively, the uses of a fiber assembly according to the invention are *ex vivo* and/or *in vivo* but without invasiveness in the mammal's body, in particular in the human's body.

## Process for manufacturing a swollen fiber assembly

The invention also relates to a process for manufacturing a swollen fiber assembly according to the invention, comprising the following steps:

a) providing at least two fibers in the dry state, said at least two fibers being made of a material comprising at least one polymer, the chains of the at least one polymer being chemically and/or physically crosslinked with each other and oriented on a macromolecular scale in the dry state, then
b) non-weaving, twisting, weaving, braiding or knitting the at least two fibers together, and/or including them within a polymeric matrix, then
c) contacting the at least two fibers, that are either non-woven, twisted, woven, braided or knitted together, or included within a polymeric matrix, with at least one swelling agent.

Preferably, the step b) is carried out without heating the at least two fibers above the glass transition temperature or the melting temperature of the oriented polymer segments nor contacting the system with a swelling agent during the assembly and/or inclusion. Alternatively, if the assembly process requires it, the heating above the glass transition temperature or the melting temperature of the oriented polymer segments or contacting the system with a swelling agent may be carried out during the assembly and/or inclusion.

The invention also relates to a process for manufacturing a swollen fiber assembly according to the invention comprising the following steps:

- providing at least two fibers in the dry state, said at least two fibers being made of a material comprising at least one polymer, the chains of the at least one polymer being chemically and/or physically crosslinked with each other and oriented on a macromolecular scale in the dry state,
- non-weaving, twisting, weaving, braiding or knitting the at least two fibers together, and/or including them within a polymeric matrix, while maintaining the assembly in a predefined shape using a frame or a mold,
- contacting the at least two fibers, that are either non-woven, twisted, woven, braided or knitted together, or included within a polymeric matrix, with at least one swelling agent.

The present invention also relates to any product obtained by the process according to the invention for manufacturing a swollen fiber assembly according to the invention.

## BRIEF DESCRIPTION OF THE DRAWINGS

**Figure 1** is a picture showing a scanning electron micrograph of a single dry PVA fiber.

**Figure 2** are graphs showing some microstructural characteristics of dry PVA fibers: **(A)** DSC thermogram during a heating and cooling cycle (exotherm down); **(B)** a 2D diffraction pattern produced by wide angle X-ray scattering. Fiber axis is vertical. White arrow indicates the diffraction peak produced by the (101) planes of PVA crystals; **(C)** the intensity profile integrated over an entire quadrant as a function of scattering angle $2\theta$ (Dark and light gray curves are the fits of the crystalline and amorphous contributions used to calculate the crystallinity); **(D)** the intensity profile integrated around the (101) diffraction peak ($19° < 2\theta < 20°$) as a function of azimuthal angle $\psi$.

**Figure 3** are pictures and graphs showing the evolution of the geometry of a single fiber during swelling in water. Pictures in **(A)** show the increase in the fiber diameter during the first 470 min of swelling. The graph in **(B)** shows the fiber diameter normalized by its initial dry diameter as a function of immersion time. The graph in **(C)** shows the fiber length normalized by its initial dry length as a function of immersion time. Insets in B and C are close-ups on the first 20

min of immersion.

**Figure 4** is a picture showing the twisting apparatus used to make twisted rope structures. 1: crank actuated by the operator; 2: yarns attached to the rotating hooks at one end; 3: twisted yarns assembly by counter rotation; 4: wire attached at the extremity of the rope; 5: weight.

**Figure 5** are drawings and pictures showing the organization of fibers in dry and swollen twisted double-helix rope assemblies of 4 yarns of 15 fibers. Drawing in **(A)** shows the geometrical parameters used to characterize the assemblies. Drawings in **(B)** illustrate the changes in structure and fiber dimensions upon immersion in a swelling agent (water). Pictures in **(C)** show some of the as-fabricated dry structures obtained for different values of twist: 1 (twist = 0.07 turns/cm), 10 (twist = 0.92 turns/cm), 18 (twist = 1.45 turns/cm), and 27 (twist = 3.44 turns/cm) Pictures in **(D)** show the same samples as in (B) after 12 h in water at 20°C.

**Figure 6** are graphs showing the yarn-rope angle $\alpha$ **(A),** the fiber-yarn angle $\beta$ **(B)** and the ratio l/L of length of fiber per length of structure **(C)** as a function of the twist in the as-fabricated dry state for double helix rope assemblies of 4 yarns of 15 fibers. Full symbols show the values measured on the as-fabricated dry structures. Open symbols show the values measured on the swollen structures after a 12 h immersion in water.

**Figure 7** are pictures and graphs showing the change in the length of the twisted double helix structures after immersion in water. The picture in **(A)** shows ten structures having various twists after 15 s of immersion. The picture in **(B)** shows the same structures after 1 h 45 min of immersion. The graph in **(C)** shows the contraction rate for all the structures as a function of their twist in the as-fabricated dry state. Light gray symbols correspond to measurements obtained with control structures made from fibers that were previously swollen and redried.

**Figure 8** are graphs showing the elongation rate of the fibers inside the swollen twisted double helix structures as a function of the ratio $l_s/L_s$ of length of fiber per length of swollen structure. **(A)** corresponds to the samples made according to the invention. **(B)** corresponds to control structures made from fibers that were previously swollen and redried.

**Figure 9** are graphs showing the tensile behavior of swollen fibers and structures. The graph **(A)** shows the force-strain curves measured on each one of the 15 swollen fibers composing a yarn. The graph in **(B)** shows the normalized tensile stiffness Klnk of the swollen twisted double helix structures as a function of the ratio $l_s/L_s$ of length of fiber per length of swollen structure (Black symbols). Light gray symbols correspond to the measured values for the control structures. Full lines are predictions given by different rope mechanics models.

**Figure 10** is a schematic representation of the fabrication process to make composite tubular structures. **(A)** A cylindrical holder was used to set the tubular shape. **(B)** the yarns were wrapped around the cylinder. **(C)** The cylinder was introduced inside a mould and the cavity was filled with PVA solution. **(D)** The mould was subjected to freeze-thaw cycles. **(E)** the composite tube was removed from the mould and the inner cylinder was removed from the tube. **(F)** the composite tube was left to equilibrate in water for a week.

**Figure 11** is a series of pictures showing a knitted fiber assembly according to the invention in the form of a tube. The fiber assembly is shown in the dry state after fabrication **(A-B),** after swelling during 7 days in water at 20°C **(C-D)** and after longitudinal cuts performed on the swollen structure revealing the presence of non-zero tensile residual stresses oriented along the axis of the tube **(E).**

**Figure 12** is a series of pictures showing a braided fiber assembly according to the invention in the form of a tube. The fiber assembly is shown in the dry state after fabrication **(A-B),** after swelling during 7 days in water at 20°C **(C-D)** and after a longitudinal cut performed on the swollen structure revealing the presence of non-zero tensile residual stresses oriented both circumferentially and along the axis of the tube **(E).**

**Figure 13** is a schematic representation of the different composite tubular structures produced. **(A)** control structure made with 2 layers of yarns previously swollen and redried. **(B)** structure made with 2 layers of yarns wrapped perpendicularly to the tube axis. **(C)** structure made with 2 layers of yarns wrapped at 45° angle relative to the tube axis. **(D)** same as (B) but with 6 layers of yarns. **(E)** same as (B) but with 12 layers of yarns. **(F)** schematic representation of the three concentric layers composing the aorta.

**Figure 14** are pictures showing ring sections taken from the composite tubes depicted in Figure 12. Composite tubes

**(A-E)** are compared to a dissected pig aorta **(F).** Pictures in the top row show the ring sections after 7 days in water at 20°C. Pictures in the middle row show the ring sections a few seconds after a cut. Pictures in the bottom row show the same ring sections 8 hours after the cut.

**Figure 15** are drawings illustrating the principle of the invention: **(A)** dimensional changes of a fiber after contact with a swelling agent, **(B)** dimensional changes of a fiber inside an assembly according to the invention after contact with a swelling agent.

## EXAMPLES

The present invention is further illustrated by the following examples.

Example 1: Preparation of yarns comprising 15 fibers and physical characterization of said fibers

### Materials and Methods

Yarns comprising 15 fibers made of PVA were produced by a dry spinning process:

- an aqueous solution of PVA having a hydrolysis degree greater than 95% was injected through a spinneret;
- once the solution has passed through the spinneret, the PVA fibers being formed were subjected to a flow of air or other hot gas to evaporate the swelling agent and solidify the fibers;
- the resulting fibers were then caught by a recovery roller and fed to the rest of the spinning line, where 15 fibers were slightly twisted together to form one yarn, with a twist of 1.25 turns/cm.

Linear density measurements were made by weighing given lengths (100 mm) of 5 dry fibers using a precision balance (PI-214 Denver Instrument precision balance).

The geometry of the fibers was characterized by scanning electron microscopy (SEM) using a Quattro Environmental Scanning Electron Microscope (ThermoFischer Scientific) operating under an accelerating voltage of 5 kV and a beam current of 46 pA. Samples were held on a conductive copper/nickel tape and coated with a 5 nm Pd layer using a Cressington 108 sputter coater (Cressington Scientific Instruments Ltd.), operated at 30pA for 30 s. To visualize the cross-section, some fibers were cut using a razor blade.

The average diameter of the dry fibers was measured from images of 5 different regions of 5 different fibers taken using an optical microscope. A Sobel filter was applied to the images to find the edges of the fiber. The diameter was computed as the ratio of the area that the fiber takes on the image and the principal axis of this area.

The crystallinity and melting temperature were measured by differential scanning calorimetry (DSC) on a Q2000 instrument (TA instrument). Dry fiber samples weighing between 2 and 3 mg were cut and sealed in aluminum crucibles. Thermograms were obtained during heating at 10°C/min from 0°C to 250°C and then cooling at the same rate from 250°C to 0°C. The melting temperature and the crystallinity were extracted from the thermograms. The crystallinity (Xc) is given by $Xc = \Delta H / \Delta Hc$ where $\Delta H$ is the enthalpy of fusion which corresponds to the area under the melting peak of the sample and $\Delta Hc$ is the enthalpy of fusion of the pure PVA crystal ($\Delta Hc$ = 138.6 J/g) [Peppas, N. A. et al. "Crystallization kinetics of poly(vinyl alcohol)" Journal of applied polymer science vol. 27 (1982): 4787-4797]. The presence of residual water in the fibers is estimated by considering the enthalpy of vaporization of water $\Delta Hv$ = 2258 J/g.

The crystallinity and the orientation rate of the fibers in the dry state were measured by analyzing the fibers by wide angle X-ray scattering (WAXS). These measurements were performed on a Nano-inXider. The fibers were aligned parallel to each other and positioned on a cardboard support inserted into the sample holder of the Nano-inXider. Acquisitions with different sample orientations were performed and then reconstructed into a 2D image with an azimuthal domain of 180°. The angular step used was $\Delta\Psi$= 18°, thus 11 positions were analyzed with a time of 900 seconds per position.

The longitudinal contraction and radial swelling of individual fibers upon the first contact with water were characterized by immersing the dry fibers for 8 hours in water at 20°C and measuring the variation in length and diameter as a function of immersion time. For that, a fiber was held between the clamps of a 10kN Allround testing machine (ZwickRoell) equipped with a 10 N load cell (Xforce P) and a glass basin to allow testing in water. The fiber was initially held straight. The length between the clamps was measured to determine the initial length of the fiber. A picture of the initial diameter was taken using a high-speed digital camera (Basler acA2040 - 180km, Basler AG) equipped with a microscope objective 12.5:1, 1.5X FL (35-08-13-000 Optem Fusion, Qioptiq SAS). After these initial pictures, clamps were approached to loosen the fibre. Time zero was set when the water basin was raised to immerse the fibre. Every few seconds (10 at the beginning of the experiment, a few minutes at the end), the fibre was momentarily put under a small tensile load of 0.02 N, and a measure of the length and a picture were taken. Thereafter, the fibre was loosened again until the next measure. A

Sobel filter was applied to images to find the edges of the fibre. The diameter was computed as the ratio of the area that the fibre takes on the image and the principal axis of this area.

The tensile behavior of individual fibers both in the dry state and after swelling to equilibrium in water at 20°C were characterized using a 10 kN Allround testing machine (ZwickRoell) equipped with a 10 N load cell (Xforce P) and a testing chamber allowing testing in water. For fibres in the dry state, five fibres with nominal length of 250 mm were tested to rupture at a strain rate of 10%/min. The deformation of the fibres was calculated from the displacement of the clamps given by the machine. The stiffness of the fibers was calculated from the slope of force-deformation curves between 0.05 and 0.12 N. For swollen fibers, the fifteen fibres that make up a yarn were separated and immersed in water for 12 hours, and subsequently tested in tension in water. The fibres were tested in a 0 to 0.4 N cycle at a strain rate of 20%/min. No pre-cycling was applied. Deformation measurements calculated from machine displacement were found to be no different from those calculated from image correlation. Fifteen force-deformation curves were obtained. An average curve was calculated by fitting with a $4^{th}$ order polynomial function between 0 and 40% deformation. 95% confidence intervals were calculated.

## Results

The average diameter of the dry fibers composing the yarn is $80\pm10$ $\mu$m, as shown by SEM image in **Figure 1.** The linear density of the yarns (each yarn comprising 15 fibers) is $81\pm1$ mg/m ($810\pm10$ dtex), thus the linear density per fiber is $5\pm1$ mg/m ($50\pm10$ dtex).

The crystallite melting peak is at 233°C for an enthalpy of fusion of $\Delta$H=82 J/g, i.e. a crystallinity of $60\pm10$ % as measured by DSC **(Figure 2A).** In addition, the water vaporization peak is around 100°C with a total water vaporization enthalpy of $\Delta$H=84 J/g. This corresponds to a presence of 3%(g/g) water in the sample coming from the water vapor present in the air. The glass transition temperature $T_g$ measured from the cooling thermogram is 75°C.

The semi-crystalline microstructures of the fibers were characterized by wide angle X-ray diffraction. On the 2D diffraction pattern **(Figure 2B),** the peaks of the crystal planes $10\bar{1}$ and 101 are overlapping. The $10\bar{1}$ and 101 crystal planes contain the axis of the polymer chains. The appearance of these peaks perpendicular to the fiber axis indicates that the polymer chains are predominantly oriented along the fiber axis. In addition, the $2\theta$ intensity profile of the integrated diffraction patterns over an entire quadrant **(Figure 2C)** showed the expected position of the peaks of the $10\bar{1}$ and 101 planes at 19.5°, the 100 plane at 11°, and the 200 plane at 22.8°. The deconvolution of this profile allowed to determine the crystallinity which was 31%.

The macromolecular orientation of the fibers was characterized by WAXS diffraction. For that, the scattered intensity integrated in the vicinity of the amorphous, 101 and $10\bar{1}$ peaks was plotted as a function of the azimuthal angle $\psi$ where $\psi$ = 0° in the direction of the fiber **(Figure 2D).** The value of the corresponding Hermans orientation factor was $f_H = 0.75\pm0.20$. Such a value close to 1.0 indicates that the axis of the polymer chains are preferentially oriented parallel to the fiber axis.

The tensile behavior of individual dry fibers exhibits a linear part in the first 0.9% of deformation with a stiffness of $41\pm3$ N/unit strain. A yield point is reached at 0.3 N and 0.9% elongation and is followed by a plastic zone until rupture with an elongation at break of 20-30%.

When dry PVA fibres are immersed in water, they swell to form hydrogel fibres. Pictures in **Figure 3A** show the evolution of the fibre diameter during 8h in water at 20°C. During swelling, the diameter of the fibre increased while the length decreased, as indicated in **Figure 3B** and **C** showing the diameter and length normalized by their initial values in the dry state as a function of immersion time. Most of these dimensional changes take place during the first 10 minutes of swelling and they slow down strongly until reaching a plateau after about 1.5 h. At equilibrium swelling, the diameter has increased by $44\pm6$% and the length has decreased by $29\pm1$% (contraction rate) as compared to the initial dry state.

The uptake in swelling agent (water here) of the fiber was measured in weight (resp. volume) and was given by the increase in weight (resp. volume) between the state at equilibrium swelling and the as-spun dry state divided by the weight (resp. volume) of the in as-spun dry state. It was measured to be $50\pm10$ % in weight and $32\pm7$ % in volume.

The crosslinking of the polymer composing the fibers was evidenced by the non-dissolution of the fibers in water at 37°C for more than one month and at room temperature for more than five years. Physical crosslinking was evidenced by the complete dissolution of the fibers in water upon heating above melting temperatures of PVA crystallites in water (95°C).

The tensile behavior of the individual fibers after free swelling at equilibrium in water at 20°C for 12 hours was characterized. **Figure 9A** shows 15 loading curves for each one of the 15 fibres composing a yarn. The non-linear response is noticeable with an increase in the stiffness of the fiber with increasing tensile strain. A fourth degree polynomial fit was used to calculate the stiffness as a function of the tensile strain.

Example 2: Fabrication of some fiber assemblies having twisted rope structures according to the invention

**Materials and Methods**

The assemblies were made using the S-twisted yarns of example 1 (1 yarn = 15 fibers) or using Z-twisted strands having a pitch of 2 cm/turn which were themselves composed of 15 S-twisted yarns of example 1 (1 strand = 15 yarns = 165 fibers). A twisting device comprising a wheel (comprising 16 hooks distributed in a circular manner on a plate) and a square (consisting of a hook connected to a swivel which can be locked or free to rotate to allow the twisting of the structure) was used for manufacturing fiber assemblies according to the invention, as shown in **Figure 4.** The direction of rotation is chosen to accentuate the twist already present on the yarns and strands. To do this, the crank must be turned clockwise for yarns with an S helix and counterclockwise for strands with a Z helix.

Black, water-resistant, permanent ink was used to dye some of the fibers and visualise them inside some of the rope structures (Staedtler Lumocolor® black, Staedtler Mars). For this, the yarn was passed through a cotton ball impregnated with the ink with the help of a sewing needle. The absence of effect of the dye on the yarn swelling behavior and tensile response was verified on two dyed yarns.

**Results**

Twenty-nine structures consisting of twisted double helix assemblies of 4 yarns (60 fibers) were obtained as depicted in **Figure 5A.** These structures were made from 4 parallel yarns separated on 4 hooks and twisted together with different twisting rates ranging from 0.01 tr/cm to 2.97 tr/cm. Optical micrographs of some of the twenty-nine structures in the dry state are shown in **Figure 5C,** where one of the yarns was dyed in black prior to assembly.

As a control, twelve structures consisting of twisted double helix assemblies of 4 yarns (60 fibers) were also produced with the same method as above but using yarns that had been previously swollen to equilibrium in water at 20°C for 12 h to release the frozen orientation and subsequently re-dried in air at 20°C for a week. Twisting rates were varied from 0.01 tr/cm to 3.31 tr/cm. These samples are referred to as controls.

Three structures consisting of 6-strand twisted assemblies (1350 fibers) were obtained. The first structure was made using 2 groups of 3 strands per hook and twisting them. The second structure was made in the same way but this time by distributing the strands in 3 groups of 2 strands per hook. The third structure was made by first twisting strands by 2 (1 strand per hook) to make bundles; three of these 2-strand bundles were then twisted together to form the structure.

Example 3: Physical characterization of some twisted fiber assemblies according to the invention

**Materials and Methods**

The twenty-nine rope structures according to the invention obtained in example 2 consisting of twisted double helix assemblies of 4 yarns (60 fibers) and the twelve corresponding control structures were studied. Two portions were cut from each sample: one 200 mm long portion was used for geometrical characterization and mechanical testing, and one 100 mm long portion was used for destructive testing. For each portion, a knot was tied at each end to prevent fraying. The 200 mm specimens were weighted using the precision balance.

The geometry of each dry structure was characterized by taking a picture with a digital camera (Nikon D5000) equipped with a 60 mm objective (1:2.8 D AF Micro Nikkor). From these images, the lay length (cm/turn) was computed as the length of the rope divided by the number of turns of one yarn around the axis of the twisted rope structure. The twist (turns/cm) was defined as the inverse of the lay length. The angle that each yarn makes with the rope axis ($\alpha$) and the angle that each fibre makes with its yarn axis ($\beta$) were measured manually on each image, as indicated in **Figure 5A.** For angle $\alpha$, a mean value was calculated out of 6 to 12 measurements per specimen. For angle $\beta$, a mean value was calculated out of 6 measurements per specimen.

Destructive tests were performed using the 100 mm specimens to validate the geometrical measurements on dry structures. For that, the total length of each rope was first measured. It was then frayed by separating it into its four strands, the lengths of which were measured. Then, each strand was frayed and separated into fibres, the length of which were again measured. These experimental measurements were compared to the values calculated from angles $\alpha$ and $\beta$.

The swelling of the structures in contact with a swelling agent was characterized by immersing the 200 mm specimens in distilled water at 20°C for 12 hours. To study the swelling kinetics, two small marks of India ink mixed with epoxy resin were made at each end of the ropes. During the first 100 minutes of immersion, some of the structures were arranged vertically in a frame inside a transparent glass container. Their upper end was fixed to the frame and a one-gram weight was attached to the lower end. Images were taken every 15 seconds to determine the contraction kinetics of the ropes. The contraction rate was calculated from the images using the length between the knots of the dry and swollen specimens. After this time, the weight was unhooked, and the ropes were left to contract freely along with the rest of the

specimens for the remainder of the 12 hours.

The mass and length of the swollen specimens were measured using the precision balance and a calliper (Pro-Max electronic digital calliper). The lay length and the angles $\alpha$ and $\beta$ were measured with the same method as described above for the dry structures. The diameter of the yarns inside the swollen ropes was also measured from the images, using the software ImageJ.

The tensile behavior of the dry and swollen structures were characterized along the structure length using a 10 kN Allround testing machine (ZwickRoell) equipped with a 10 N load cell (Xforce P). For the dry structures, testing was performed in air at 20°C and stretching-unstretching cycles were applied at 10%/min between 0.05 N and 8 N. For the swollen structures, testing was performed in water at 20°C and stretching-unstretching cycles were applied at 20%/min between 0.05 N and 2 N. For each sample, four pre-cycles were applied, and the last cycle was recorded. The tests were recorded using the high-speed camera equipped with a 1:2.0, 16mm ED AS wide-angle lens (Samyang Optics). Images were analyzed using the digital image correlation software Vic-2D (Correlated Solutions Inc.) to measure the tensile strain. Force-strain curves were obtained and the stiffness of the ropes was calculated as the slope of the curve between 3 and 7 N for dry structures and between 0.3 and 1 N for swollen structures.

**Results**

For a rope structure of length L, the length of one fibre 1 in the structure was calculated from the values of angles $\alpha$ and $\beta$ using a geometrical model of a double-helix structure, as follows:

$$l = L / (\cos\alpha * \cos\beta) \qquad \text{(Equation 1)}$$

In the dry state, for each structure, the angles $\alpha$ and $\beta$ were measured as shown in **Figure 6A** and **B**, respectively. The angle $\alpha$ increased rather linearly with the increasing twist from 0° to about 30°, while the angle $\beta$ remained fairly constant (7°) for twist values up to 1.2 turns/cm and then increased linearly up to about 25°. **Figure 6C** shows the length of fibre per unit length of structure (1/L) as calculated from measured angles of $\alpha$ and $\beta$ using Equation (1). The I/L ratio increased non-linearly with the rope twist. It remained almost constant (1.02) for twist values lower than 1.0 tum/cm and then it increased linearly up to 1.25 for a maximum twist of 2.91 turns/cm. A good agreement is obtained between the values of I/L calculated from angles $\alpha$ and $\beta$ and the values of I/L measured by destructive testing, which confirms that the proposed non-destructive image analysis method is appropriate to assess the length of the fibers within the structures.

The dry structures were immersed in water to induce swelling and contraction of the PVA fibres within the double helix ropes, as depicted in **Figure 5B. Figure7A** and B show pictures of 10 structures having a twist ranging from 0.03 to 2.91 after 15 seconds and 105 min of immersion in water, respectively. For all ten ropes, a contraction started after about 10 min of immersion. Like for the individual fibres, most of the contraction was achieved within the first 20 min of immersion but contraction slowly continued during the first hour of immersion until a stable length was reached for all the structures after 90 min.

The contraction rate was defined as ($1 - L_s/L_d$), where $L_d$ is the initial dry length of the rope and $L_s$ is the length of the swollen structure at equilibrium swelling. **Figure 7C** shows the contraction of all the structures as a function of the rope twist applied during fabrication. For the least twisted structures (rope twist close to zero), the contraction rate was close to 30%, like the contraction rate of individual fibres (29%). For rope twists between 0 and 1.2, the contraction rate increased up to 40%. For rope twists greater than 1.2, the contraction rate decreased with rope twist reaching a value of less than 30% for the highest rope twists close to 3. This decrease in the contraction rate above twist 1.2 indicates the onset of friction between the fibres within the structure, sufficient to prevent the fibres from contracting completely.

The same swelling experiment was performed with control ropes in which the fibres have been previously swollen and re-dried. As shown in **Figure 7C,** the control ropes did not contract, but elongated by about 8%, which is consistent with the behaviour of the individual swollen and re-dried fibres where the macromolecular orientation has been released.

The geometrical changes induced by swelling in the twisted double helix structures were characterized by measuring the angles $\alpha$ and $\beta$ on images like the 4 samples in **Figure 5D.** The angle $\alpha$ between the yarn axis and the rope axis was larger in the swollen structures than in the dry structures (full symbols in **Figure 6A).** The angle $\beta$ between the fibre axis and the yarn axis also increased with swelling (full symbols in **Figure 6B).** The fibre length per unit length of the twisted double helix structure (1/L) in the swollen structures was calculated from the measured values of $\alpha$ and $\beta$ using the geometrical model validated in the dry structures (Equation 1). For structures having a twist greater than 0.3, the I/L ratio was found to increase with swelling (full symbols in **Figure 6C).**

In the control structures, the angles $\alpha$ and $\beta$ barely changed with swelling. The fibre length per unit rope length in the control structures was similar before and after swelling. The range of I/L ratio values of the control structures (1-1.6) was similar to that of the non-pre-relaxed structures.

The presence of residual strains and stresses in the swollen structures was characterized by calculating the

elongation rate of the fibres inside the swollen ropes. Consider a swollen fibre having a length $l_s$ after swelling within a swollen structure. This fibre would have had a length $l_{eq}$ if it was free to swell and reach equilibrium swelling outside of a structure. The elongation rate $\gamma$ of this fibre within the swollen double helix assembly is given by :

$$\gamma = l_s / l_{eq} \qquad \text{(Equation 2)}$$

Equation (2) can be reformulated using measurable quantities as follows:

$$\gamma = [(1 - \Lambda) \cdot (\cos \alpha_d \cdot \cos \beta_d)] / [(1 - \lambda) \cdot (\cos\alpha_s \cdot \cos\beta_s)] \qquad \text{(Equation 3)}$$

where $\alpha_d$ and $\beta_d$ are the angles of the dry double helix assemblies, $\alpha_s$ and $\beta_s$ are the angles of the swollen double helix assemblies, A is the contraction rate of the structure ($1 - L_s/L_d$) and $\lambda$ is the contraction rate of the individual fiber ($1 - l_{eq}/l_d$) (29%).

**Figure 8A** shows the elongation rate of the fibres inside the swollen structures as a function of the ratio $l_s/L_s$ for all the studied samples. The ratio $l_s/L_s$ gives an indication of the amount of fibre-fibre friction within a swollen structure. Indeed, the greater $l_s/L_s$ is, the more fibre-fibre contacts are present in the structure. Two regimes were observed.

For $l_s/L_s < 1.1$, no noticeable tensile strain was applied to the fibers. Slippage occurred between the fibres and the fibre-on-fibre friction was not enough to prevent the fibres to contract and relax completely during swelling.

For $l_s/L_s > 1.1$, the fibres were stretched within the swollen structures and the value of the elongation rate increased linearly with $l_s/L_s$, reaching 135% in the most twisted double helix assemblies. The presence of these residual stresses can be explained by the fact that the fibres were more tightly packed in those structures and fibre-on-fibre friction prevented their full contraction during swelling.

**Figure 8B** shows the measured values of the elongation rate in the case of the control structures made with swollen-redried fibres. For all the studied range of twist values, no significant tensile strain was measured indicating that these structures have no residual tensile strains. The values of elongation rate greater than 100% can be explained by the uncertainty of the values of $\alpha$ and $\beta$, which was larger than for the structures produced according to the invention.

**Figure 9B** shows the normalized tensile stiffness Klnk of the swollen structures as a function of the ratio $l_s/L_s$. On this graph, the tensile stiffness of the swollen structures K is normalized by nk where n is the number of fibers in the structures (60) and k is the tensile stiffness of one swollen fiber (0.2 N/unit strain). In other words, nk is the stiffness of a structure where all the fibers are parallel to each other. Several regimes can be distinguished.

For $l_s/L_s < 1.2$, the stiffness of the structure decreases with the ratio $l_s/L_s$. This decay is well described by a full slip model of elastic twisted rope where friction between the fibers is negligible.

For $1.2 < l_s/L_s < 1.4$, the stiffness of the structure remains fairly constant. This behavior is well described by a no-slip model where sliding between fibers is completely impeded due to friction between the fibers.

For $l_s/L_s > 1.4$, the stiffness of the structure increases with the ratio $l_s/L_s$. This remarkable behavior cannot be explained by the no-slip model and it was not observed in the control structures. This increase in stiffness is attributed to the stretched state of the fibers within the structures according to the invention. Since the swollen fibers have a non-linear tensile behavior (see **Figure 9A),** their stiffness increases with their elongation rate. For $l_s/L_s > 1.4$, the values of elongation rates exceed 110% and the stiffness of the fibers is increased by a factor 1.5 to 4. A no-slip model modified to account for this stiffening of the fibers as a function of the elongation rate also predicts an increase in the stiffness of the structures according to the invention (model no slip k($\varepsilon$)). These results show that the controlled introduction of residual stresses through fibre shrinkage provides a way to tailor the mechanical response of fibre assemblies. It was possible to design and fabricate twisted double helix assemblies which stiffness increases with twist, which is impossible to achieve in standard double helix assemblies.

<u>Example 4: Manufacture of some fiber assemblies having tubular shapes according to the invention</u>

**Materials and Methods**

Tubular assemblies were made with the yarns of example 1 (1 yarn = 15 fibers) or with strands twisted having a pitch of 2 cm/turn from yarns of example 1 (1 strand = 15 yarns = 165 fibers).

Knitted tubular structures were fabricated by assembling the dry strands with a manual knitting mill Prym 624181 Comfort Twist Knitting Mill (Prym).

Woven tubular structures were fabricated by weaving dry strands in a custom-made circular loom consisting of two 50 mm diameter circular Teflon plates with a thickness of 10 mm, arranged concentrically and spaced 70 mm apart by a central threaded rod. At the periphery of each of the plates were eighteen, 5 mm indentations arranged radially at equal angular intervals. The vertical weft of the fabric was made by passing 18 strands through the indentations of the Teflon

plates, in such way that they were arranged circumferentially and parallel to the axis of the central rod. To complete the weft, a rope was passed alternately between the vertical ropes and almost perpendicular to them, forming a spiral.

Tubular structures with the fiber assembly included within a polymer matrix were fabricated using a custom-made stainless-steel mold. The mould consists of a 220x60x40mm stainless-steel block that can be disassembled into two pieces to allow unmoulding of the casting. Inside is a 140x25mm diameter cylindrical cavity, blind at one end and open at the other end to the surface of the block, through which the liquid can be poured. Concentric to this cylindrical cavity there is a cylindrical core of 20 mm diameter as depicted in **Figure 10A.** This core is made up of two parts to facilitate the unmoulding of the tube without breaking.

To make the mesh of the composite tubes, a yarn was wound around the core of the mould **(Figure 10B).** By varying the number of layers and the angle of the yarn to the mould axis. PVA aqueous solution at 10 wt% was prepared using PVA powder (Mw = 89-98 kg/mol, hydrolysis 99+%) (Sigma Aldrich) and ultrapure water. The bottom part of the mould was sealed and a PVA aqueous solution at 10% weight was poured from an aperture at the top of the mould **(Figure 10C).** The mould was degassed at room temperature in a vacuum chamber (Binder VD23 Vacuum) for two hours. To obtain the gel matrix, the gel inside the mould was subjected to 3 freezing-thawing cycles of 12 hours each using a -18°C freezer **(Figure 10D).** After this, the hollow tubes were extracted from the mould **(Figure 10E)** and kept in distilled water for 7 days until testing

**(Figure 10F).**

**Results**

Dry knitted tubular structures having a 1 cm external diameter and 9 cm length were obtained as shown in **Figure 11A.** The spatial organisation of the fibers in the dry knitted structure is shown in **Figure 11B** with a characteristic length $h_d$ of the dry knitted pattern of 6 mm.

Dry woven tubular structures having a 3-4 cm external diameter and 5-6 cm length were obtained as shown in **Figure 12A.** The spatial organisation of the fibers in the dry woven structure is shown in **Figure 12B** with a characteristic length $h_d$ of the woven pattern of 5 mm.

Several tubular structures with the fiber assembly included within a polymer matrix were produced by varying the number of yarn layers and the angle between the yarn and the axis of the inner cylinder of the mold, as depicted in **Figure 13** and described as follows: three structures composed of 2, 6 and 12 layers of yarns wrapped perpendicularly to the tube axis **(Figure 13B, D** and **E,** respectively); one structure composed of 2 layers of yarns wrapped at a 45° angle with the tube axis **(Figure 13C);** one control structure composed of 2 layers of yarns wrapped perpendicularly to the tube axis using yarns that were previously swollen and dried to remove the ability of the fibers to contract upon contact with a swelling agent (water) **(Figure 13A).** Another control structure was obtained by making a tube with the polymer matrix only (not shown).

<u>Example 5: Physical characterization of some fiber assemblies having tubular shapes according to the invention</u>

**Materials and Methods**

The knitted, woven and composite molded tubular assemblies obtained in example 4 were studied.

For comparison, a 100 mm long section of thoracic aorta was harvested post-mortem from a healthy pig that was euthanized for a reason other than this experiment. The procedure was carried out by an authorized veterinarian (Ecole Nationale Véterinaire d'Alfort, France). The sample was kept in 0.9% m/v saline until opening angle tests 48 hours later.

The dimensions and shape of the dry knitted and woven tubular assemblies as well as the spatial arrangement of the fibers within these assemblies were characterized by optical micrographs.

All the tubular structures were left to swell freely in distilled water at 20°C for 7 days. Pictures of the swollen structures were taken, and their dimensions and weight were measured.

The presence of oriented residual stresses in woven and knitted structures was characterized in two ways. In a non-destructive way, the elongation rate of the fibers within the swollen structures was assessed from images of the dry and wet structures. In a destructive way, the tubes were cut longitudinally using a scalpel and pictures were taken to record the deformation underwent by the tube following the cut.

The presence of circumferential residual stresses in the composite molded tubular structures and in the explanted pig aorta were characterized by opening angle experiments. For each tube, 2-mm high ring-shaped specimens were collected. The rings were placed in a large petri dish filled with distilled water (tubular structures) or with 0.9% w/v saline (aorta). A radial cut was made through the ring wall with a surgical scalpel, and was left to deform freely while being recorded. The evolution of the opening angle was monitored for eight hours after the cut by periodic imaging.

**Results**

Different textile assembly techniques were used to tune the orientation of the residual stresses inside the structures. Structures with a radial gradient in residual stresses were obtained using the knitting assembly method. The dry knitted tubes **(Figure 11A)** contracted after swelling in water **(Figure 11D)** with a contraction rate of 15±5% along the tube axis. The knitted pattern can be distinguished into two parts: a part exposed to the ouside of the tube where the fibres are mostly oriented along the tube axis and a part exposed to the inside of the tube where they are oriented circumferentially. Upon swelling, the fibres in the outer part put the fibres in the inner part under compression. The swollen structure was tightly compacted **(Figure 11C)** and it can be approximated by a bilayer composed by an outer layer in axial tension and an inner layer in axial compression. By measuring the height of the wales before and after swelling ($h_d$ and $h_s$ in **Figure 11B** and **C**), an elongation rate of the fibres of 124±3% was calculated in the outer part of the structure. The presence of these residual stresses was evidenced by sectioning the courses using the strip curling technique. For that, a wale line was uncoupled from the rest of the structure that caused the wales to spring open and curve outwards, as shown in **Figure 11E.**

Structures with residual shear stresses along the tube axis were obtained using the weaving assembly method. The dry woven structure **(Figure 12A)** contracted during immersion in water until it reached its equilibrium dimensions **(Figure 12D).** During this swelling process, the radial and circumferential components of the force exerted by the circular weft yarn when contracting were balanced by the swelling against the vertical weft yarns. Since the circular weft yarn was a continuous spiral yarn-and it was not perpendicular to the tube axis-, it exerted an axial force when contracting, which in equilibrium is compensated by the symmetry of the structure. By measuring the width of a rope loop before and after swelling ($h_d$ and $h_s$ in **Figure 12B** and **C**), an elongation rate of about 117% was calculated for the fibres composing the circular weft yarn. The presence of these residual stresses was evidenced by making a longitudinal cut. Upon cutting, the axial component of the contraction force was no longer balanced, which caused the cylinder to deform **(Figure 12E).** The circumferential and radial RSS remained balanced, as there was no change in diameter, and, unlike the knitted tube, the cylinder walls remained parallel to the axis, as shown by the point p staying on the same vertical line as point p' **(Figure 12E).**

Structures with circumferential residual stresses were obtained by making hydrogel composite tubes where the fibres are embedded in a PVA hydrogel matrix. In this case, the contraction of the fibres was impeded by friction and adhesion between the fibres and the matrix. In order to prevent the full contraction of the fibres during the fabrication, a rigid mould was used to hold the fibres until the hydrogel matrix solidified. Several tube designs were fabricated to adjust the intensity of the circumferential tensile prestrain, as illustrated in **Figure 13B**-E. In those tubes, the number of yarn layers and their orientation were varied. After equilibration in water for a week, the size of the tube changed depending on the fibre content, indicating a competition between fibre contraction and opposition of the outer hydrogel layer. **Figure 13B** (top) shows a ring of the tube with 2 layers of yarns wrapped perpendicularly to the tube axis. The inner diameter of the cylinder (17 mm) decreased from its original diameter inside the mould (20 mm). A similar contraction was observed when the fibre layers were arranged at 45° **(Figure 13C** top). By increasing the number of layers inside the tube wall, the diameter of the tubes shrunk more **(Figure 13D** and **E** top).

After seven days of equilibration in water, the control tube with no fibres (image not shown) swelled in water and increased in diameter. The other control tube made with 2 layers of pre-relaxed fibres perpendicular to the tube axis **(Figure 13A),** did not contract from its original diameter inside the mould **(Figure 14A** top).

The tensile state of the fibres within the composite tubes swollen to equilibrium was assessed by measuring the diameter $D_s$ of the stabilized structure in water. The length of the fibers forming the inner layer of the swollen tube was given by $l_s = \pi \cdot D_s$ while the length that this fiber would have had if it was free to swell and reach equilibrium swelling outside of the structure was given by $l_{eq} = \pi \cdot D_0 \cdot \lambda$ where $D_0$ is the diameter of the mould (20 mm) and $\lambda$ is the contraction rate of the individual fiber (29%). The elongation rate $\gamma$ is then given by $\gamma = l_s / l_{eq}$. For the tube with 2 layers of yarns, the elongation rate of the fibers was about 112%. In the case of the tube with 6 layers, the elongation rate was about 111%, while in the one of 12 layers, the elongation rate was 125%. For the control tube with no fibres and the control tube with relaxed fibres, the diameter of the tube swollen to equilibrium remained equal or slightly larger than the diameter of the mould.

The presence of circumferential residual stresses was evidenced by the classic opening angle experience. Pictures in **Figure 14** show ring sections of the different tubular structures before (top), just after (middle) and 8 hours after (bottom) making the radial cut. In the case of the control tube with pre-relaxed fibres **(Figure 14A),** the geometry of the ring did not change after the cut, which indicates the absence of circumferential residual stresses. For the tube with 2 layers of yarns **(Figure 14B),** the cylinder curled inwards, resulting in a negative opening angle. For the tube with 2 layers of yarns were arranged at 45° with respect to the axis of the tube **(Figure 14C),** the opening angle was nearly zero. For the tubes with 6 and 12 layers of yarns **(Figure 14D** and **E),** an immediate elastic opening was observed, followed by a slow opening attributed to the slow visco-elastic contraction of the PVA hydrogel fibers.

As a comparison, the same opening angle experiment was performed in a pig aorta, that is known to have circumferential residual stresses (**Figure 14F**). The explanted aorta rings exhibited a similar opening angle behaviour as the tubes with 12 layers of yarns. These results show the ability to reproduce the opening angle behaviour of a ring of pig

aorta using tubular structures made of synthetic biocompatible hydrogels. The fabricated structure is made of biocompatible PVA, a material already used in cardiovascular repair. The elastic modulus of the fibres used (18±5 MPa) is not far from the circumferential elastic modulus of the aorta (0.5-3 MPa). Hence, this invention could offer a way to design more biomimetic vascular grafts that incorporate residual stresses to produce more physiological blood flow and assess compliance mismatch.

## Claims

1. A dry fiber assembly comprising at least two fibers,

   the at least two fibers comprising a polymer consisting of at least two polymer chains, the at least two polymer chains being chemically or physically crosslinked with each other and oriented on a macromolecular scale in the dry state,
   the at least two fibers being non-woven, twisted, woven, braided or knitted together, or included within a polymeric matrix further comprised in the dry fiber assembly.

2. The dry fiber assembly according to claim **1,** wherein the at least two polymer chains are physically crosslinked with each other, the crosslinks being crystallites.

3. The dry fiber assembly according to claim **2,** wherein the crystallinity of the polymer is greater than 15%, preferably it ranges from 20% to 80%, more preferably it ranges from 25% to 70%, even more preferably it ranges from 30% to 65%, the crystallinity being measured by differential scanning calorimetry.

4. The dry fiber assembly according to any one of claims **1** to **3,** wherein in each of the at least two fibers, the polymer chains are oriented on a macromolecular scale in the dry state in one direction, preferably in the direction of the fiber length.

5. The dry fiber assembly according to claim **4,** wherein the polymer chains are oriented on a macromolecular scale in the dry state in one direction after quenching below the glass transition temperature during the spinning process of the at least two fibers.

6. The dry fiber assembly according to any one of claims **1** to **5,** wherein the linear density of each of the at least two fibers in the dry state is greater than $10^{-4}$ Dtex, preferably greater than 0.01 Dtex, more preferably greater than 1 Dtex, even more preferably greater than 10 Dtex.

7. The dry fiber assembly according to any one of claims **1** to **6,** wherein the polymer is selected from the group consisting of: poly(vinyl alcohol), poly(urethane), poly(ethylene glycol) and any copolymers thereof, preferably the polymer is poly(vinyl alcohol).

8. The dry fiber assembly according to claim **7,** wherein the poly(vinyl alcohol) has a mass average molar mass ranging from 5,000 g/mol to 400,000 g/mol, preferably the poly(vinyl alcohol) has a mass average molar mass $M_w$ ranging from 25,000 g/mol to 300,000 g/mol, more preferably the poly(vinyl alcohol) has a mass average molar mass $M_w$ ranging from 80,000 g/mol to 200,000 g/mol.

9. The dry fiber assembly according to any one of claims **1** to **8,** wherein the at least two fibers are non-woven, twisted, woven, braided or knitted together, preferably the at least two fibers are twisted, woven, braided or knitted together, more preferably the at least two fibers are twisted or braided together, even more preferably the at least two fibers are twisted together.

10. The dry fiber assembly according to any one of claims **1** to **8,** wherein the at least two fibers are included within a polymeric matrix, said polymeric matrix being further comprised in the fiber assembly, wherein said polymeric matrix is made of a polymer selected from the group consisting of poly(vinyl alcohol), poly(ethylene glycol), poly(propylene glycol), poly(urethane)s, poly(acrylamide)s, poly(N,N-dimethylacrylamide), poly(acrylic acid), poly(acrylate)s, poly(2-hydroxyethyl methacrylate), aliphatic poly(ester)s, poly(saccharide)s, proteins, gelatin, synthetic polypeptides, and any copolymers thereof.

11. A swollen fiber assembly obtained by contacting the dry fiber assembly according to any one of claims **1** to **10** with at

least one swelling agent.

**12.** The swollen fiber assembly according to claim **11,** wherein the weight of the at least one swelling agent represents at least 10% of the total weight of the swollen fiber assembly at room temperature, preferably the weight of the at least one swelling agent represents from 15% to 90% of the total weight of the swollen fiber assembly at room temperature, more preferably the weight of the at least one swelling agent represents from 20% to 80% of the total weight of the swollen fiber assembly at room temperature.

**13.** The swollen fiber assembly according to claim **11** or **12,** wherein the elongation rate of the at least two fibers of the swollen fiber assembly is greater than 100%, preferably it is of at least 110%.

**14.** Use of the fiber assembly according to any one of claims **1** to **13,** for the reconstruction or replacement of a soft tissue in a mammal, in particular for the replacement or reinforcement of a soft tissue selected from the group consisting of: fatty tissues, muscles, tendons, ligaments, intervertebral disks, fasciae, skin, blood vessels, lymphatic vessels, cartilage, menisci and nerves.

**15.** Process for manufacturing a swollen fiber assembly according to any one of claims **11** to **13,** comprising the following steps:

- providing at least two fibers in the dry state, said at least two fibers comprising a polymer consisting of at least two polymer chains, the at least two polymer chains being chemically or physically crosslinked with each other and oriented on a macromolecular scale in the dry state,
- unweaving, twisting, weaving, braiding or knitting the at least two fibers together, and/or including them within a polymeric matrix, and
- contacting the at least two fibers, which are either non-woven, twisted, woven, braided or knitted together, or included within a polymeric matrix, with at least one swelling agent.

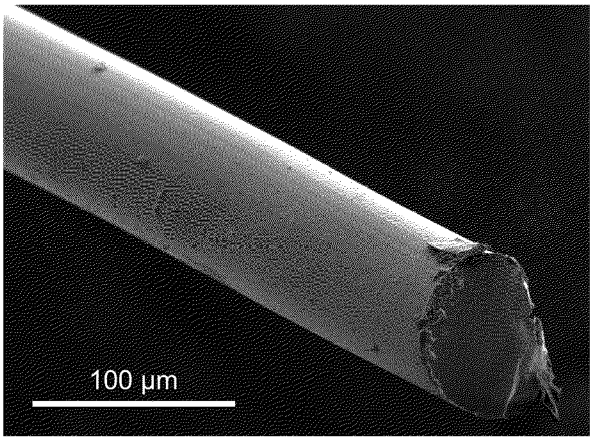
100 µm

FIG. 1

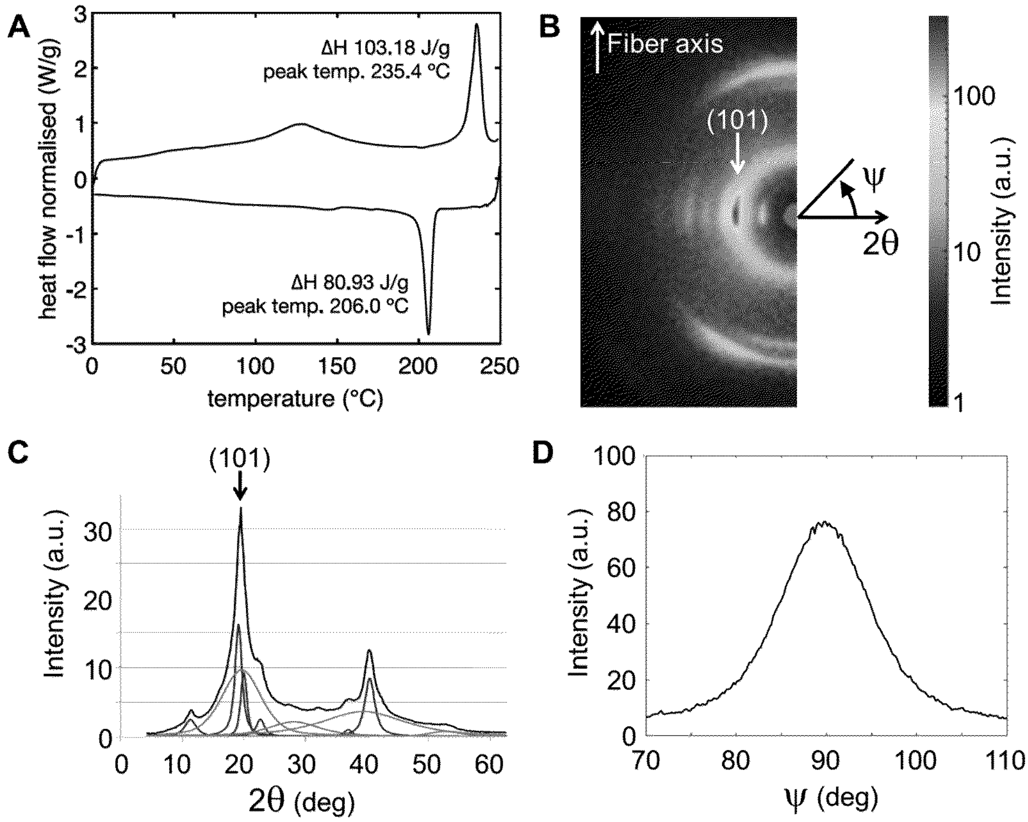
A
ΔH 103.18 J/g
peak temp. 235.4 °C
ΔH 80.93 J/g
peak temp. 206.0 °C
heat flow normalised (W/g)
temperature (°C)
B
Fiber axis
(101)
ψ
2θ
Intensity (a.u.)
C
(101)
Intensity (a.u.)
2θ (deg)
D
Intensity (a.u.)
ψ (deg)

FIG. 2

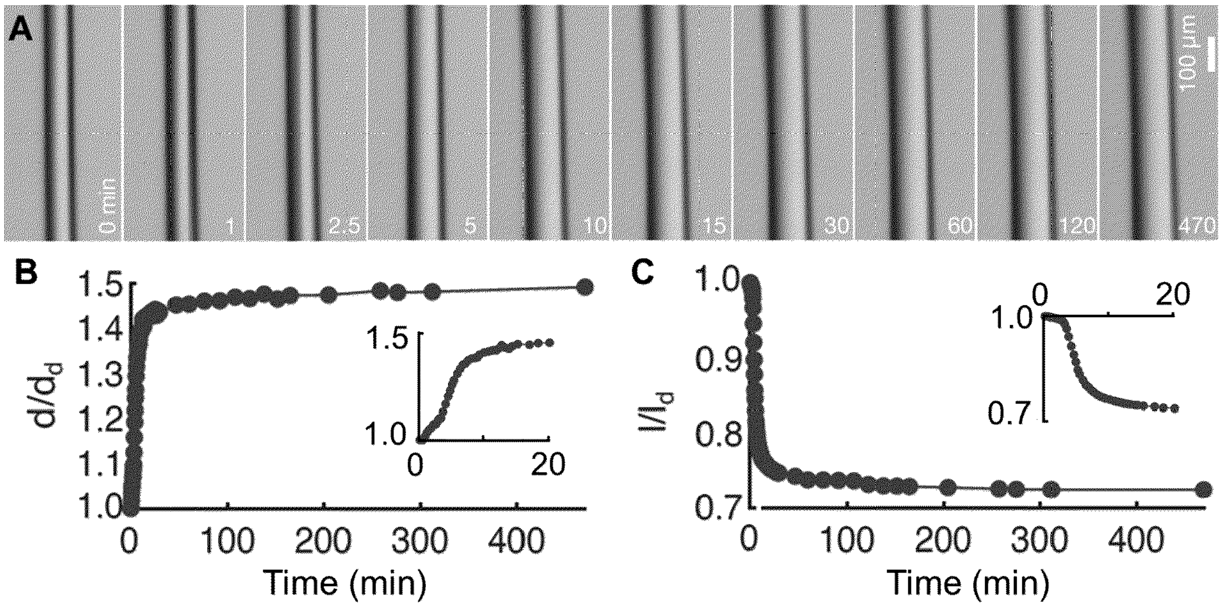
A
0 min
1
2.5
5
10
15
30
60
120
470
100 µm
B
d/d_d
1.5
1.4
1.3
1.2
1.1
1.0
0
100
200
300
400
Time (min)
1.5
1.0
0
20
C
I/I_d
1.0
0.9
0.8
0.7
0
100
200
300
400
Time (min)
0
20
1.0
0.7

FIG. 3

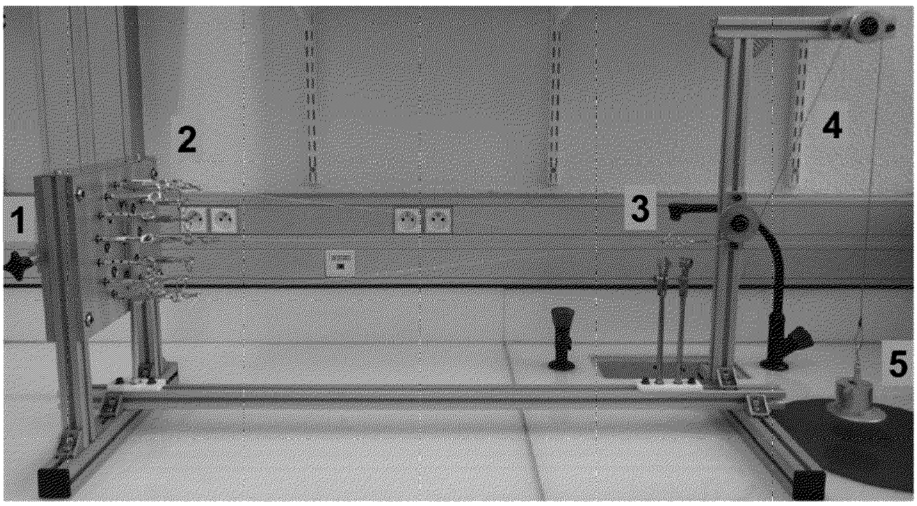
1
2
3
4
5

FIG. 4

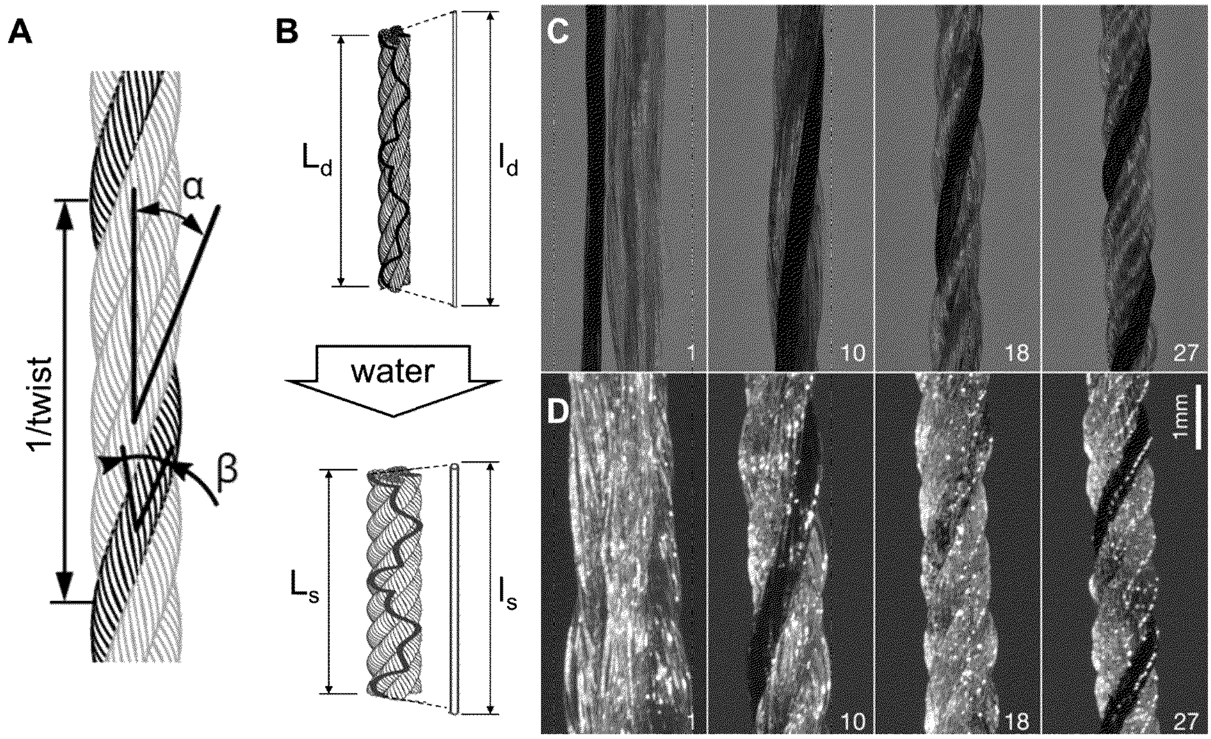
A
B
C
D
α
β
1/twist
L_d
l_d
water
L_s
l_s
1
10
18
27
1mm

FIG. 5

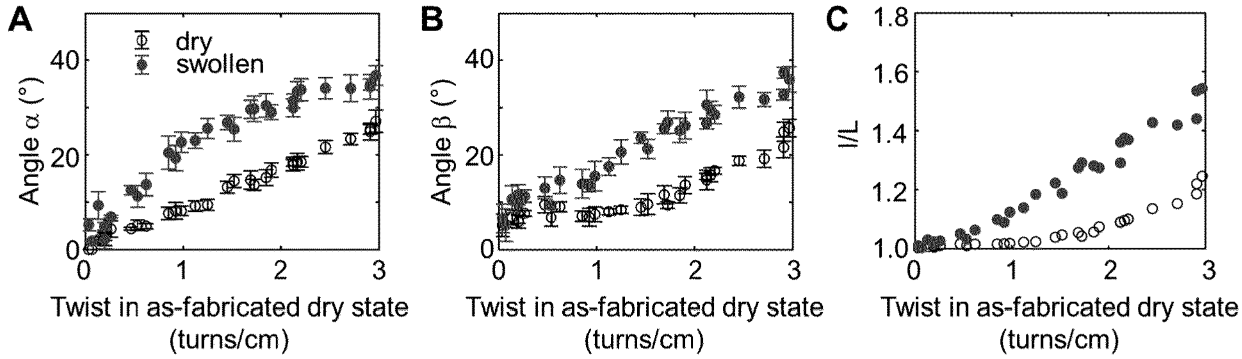
A
B
C
dry
swollen
Angle α (°)
Angle β (°)
l/L
Twist in as-fabricated dry state (turns/cm)

FIG. 6

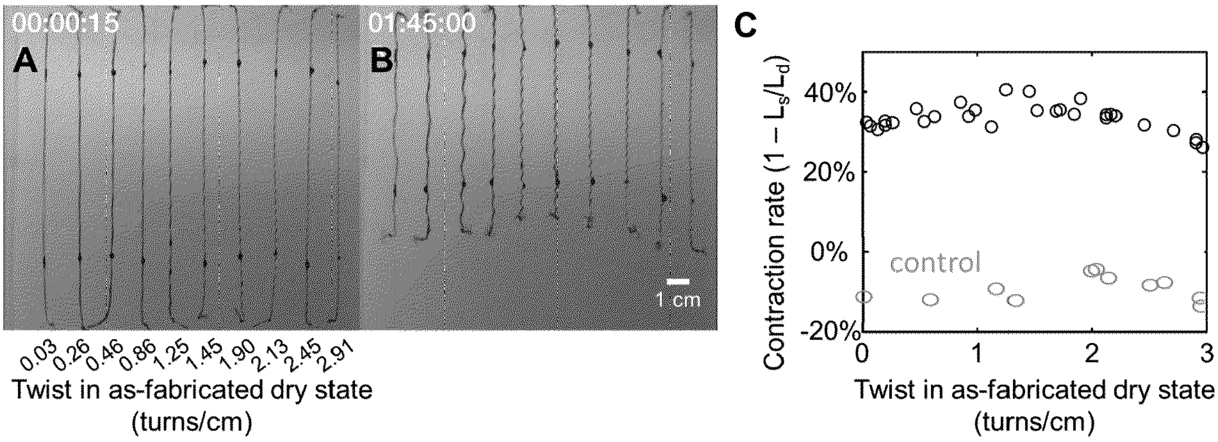


FIG. 7

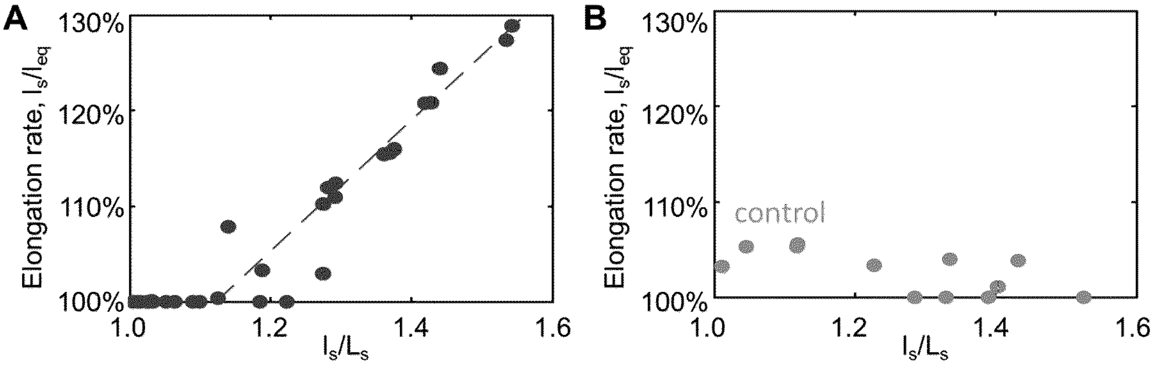


FIG. 8

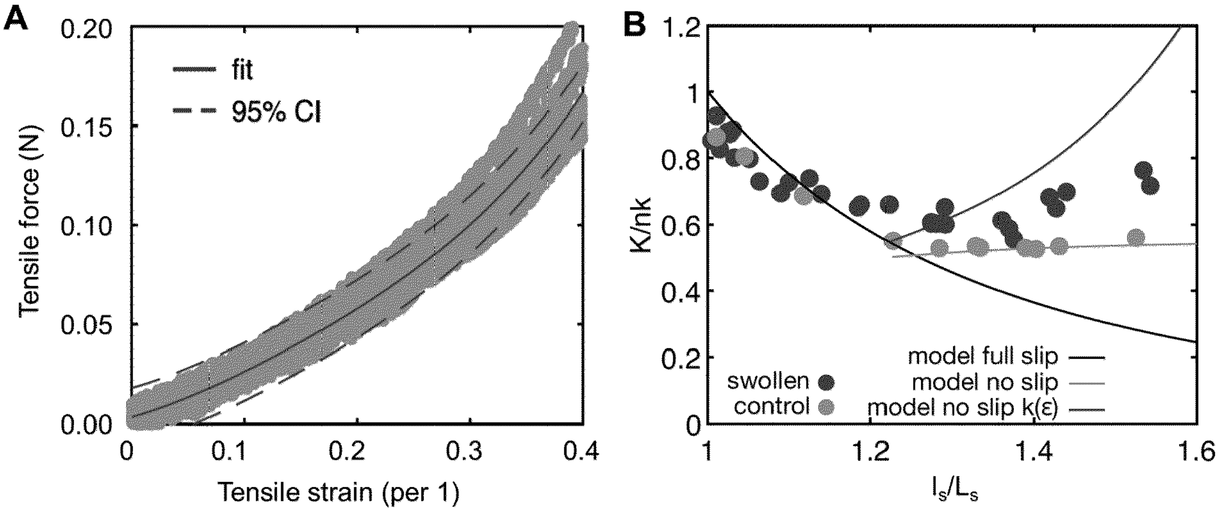


FIG. 9

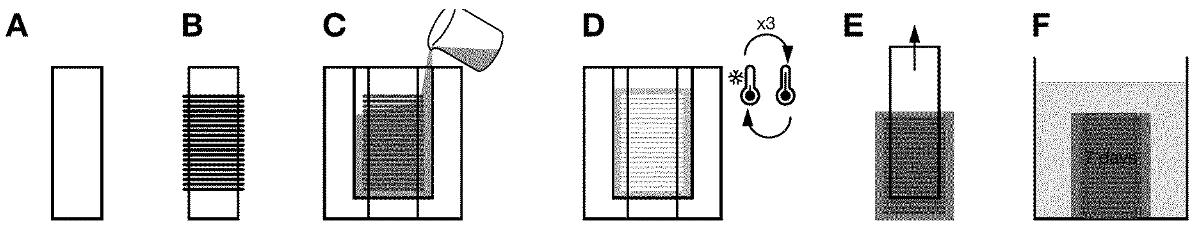


FIG. 10

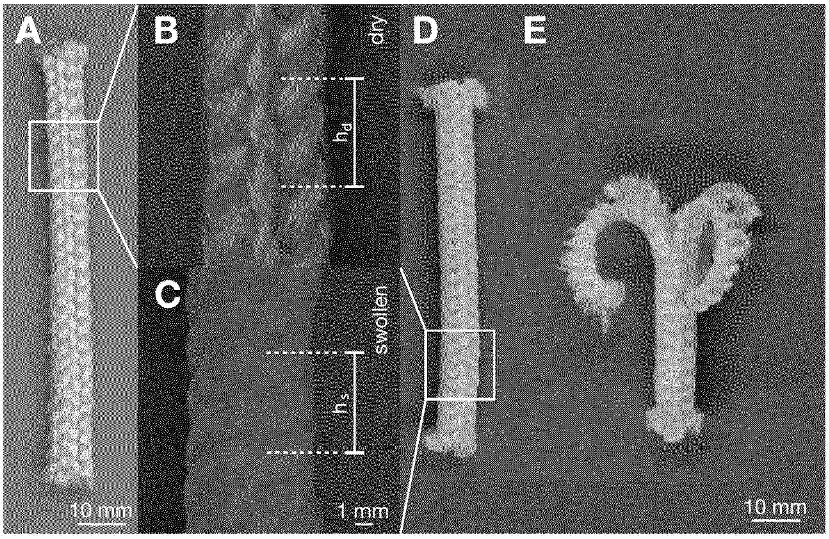


FIG. 11

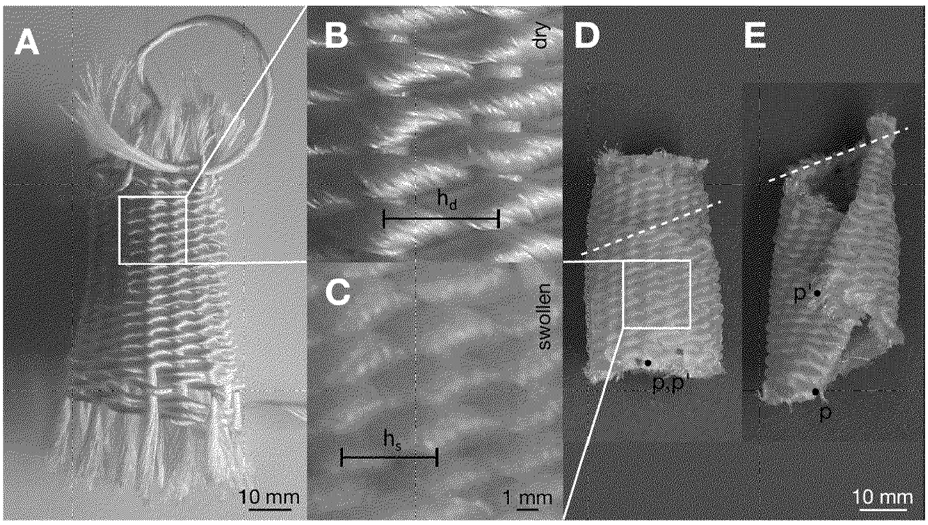


FIG. 12

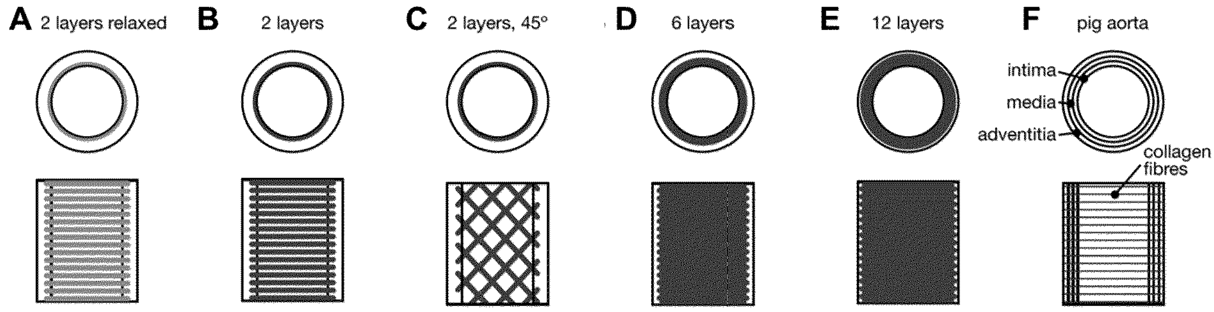


FIG. 13

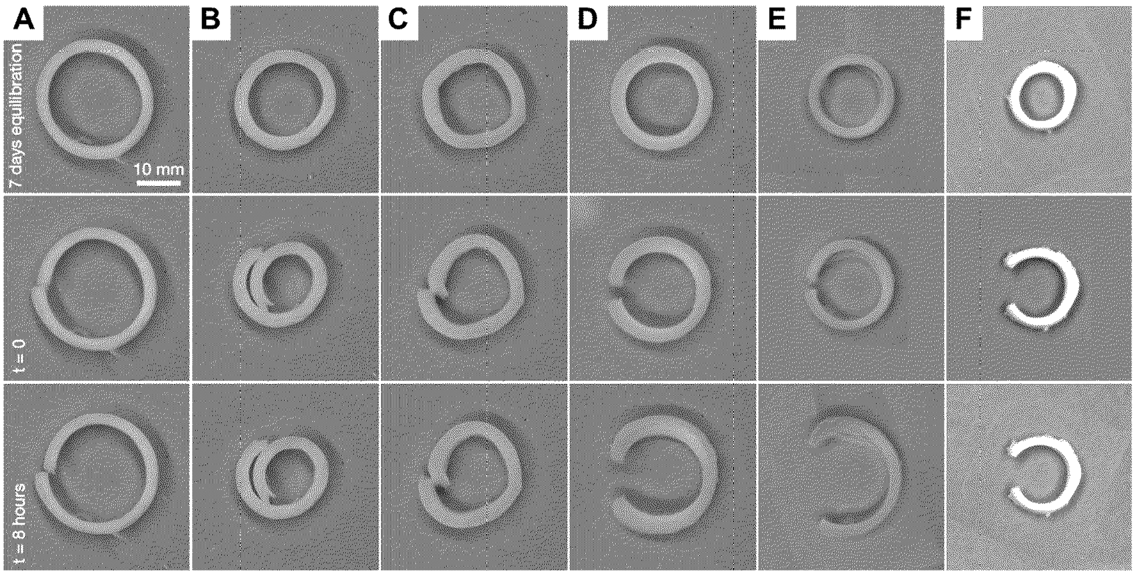


FIG. 14

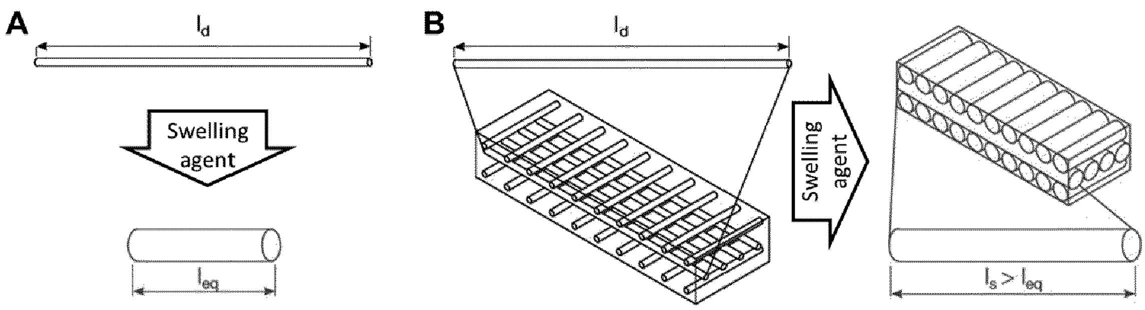


FIG. 15

Europäisches Patentamt
European Patent Office
Office européen des brevets

# EUROPEAN SEARCH REPORT

Application Number
EP 23 30 5985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2013/274415 A1 (SCHMITZ WIEBKE [DE] ET AL) 17 October 2013 (2013-10-17)<br>* paragraphs [0003], [0014], [0020] - [0025], [0029], [0032], [0043], [0045], [0074], [0088]; claims * | 1-12,14, 15<br>13 | INV.<br>D06M11/05<br>A61L27/52<br>D01F1/10<br>D01F6/14<br>D06M13/00<br>D06M15/00 |
| X<br>A | US 2007/141108 A1 (THOMAS BRIAN [US] ET AL) 21 June 2007 (2007-06-21)<br>* paragraphs [0013], [0026], [0027], [0048] - [0051]; claims * | 1-9,11, 12,14,15<br>13 | |
| | | | TECHNICAL FIELDS SEARCHED (IPC) |
| | | | D06M<br>D01F<br>A61L |

The present search report has been drawn up for all claims

1

| Place of search | Date of completion of the search | Examiner |
|---|---|---|
| The Hague | 15 December 2023 | Blas, Valérie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 30 5985

This annex lists the patent family members relating to the patent documents cited in the above-mentioned European search report. The members are as contained in the European Patent Office EDP file on
The European Patent Office is in no way liable for these particulars which are merely given for the purpose of information.

15-12-2023

| Patent document cited in search report | Publication date | Patent family member(s) | Publication date |
| --- | --- | --- | --- |
| US 2013274415 A1 | 17-10-2013 | CN 103374766 A | 30-10-2013 |
| | | DE 102012007307 A1 | 17-10-2013 |
| | | DK 2650413 T3 | 29-07-2019 |
| | | EP 2650413 A1 | 16-10-2013 |
| | | ES 2732070 T3 | 20-11-2019 |
| | | PL 2650413 T3 | 29-11-2019 |
| | | TR 201909187 T4 | 22-07-2019 |
| | | US 2013274415 A1 | 17-10-2013 |
| US 2007141108 A1 | 21-06-2007 | EP 1800701 A1 | 27-06-2007 |
| | | JP 2007167655 A | 05-07-2007 |
| | | US 2007141108 A1 | 21-06-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MICHALEK, A J et al.** Large residual strains are present in the intervertebral disc annulus fibrosus in the unloaded state. *Journal of biomechanics*, 2012, vol. 45 (7), 1227-31
- **FUNG, Y C.** What are the residual stresses doing in our blood vessels?. *Annals of biomedical engineering*, 1991, vol. 19 (3), 237-49
- **KOLBUK et al.** Optical birefringence and molecular orientation of electrospun polycaprolactone fibers by polarizing-interference microscopy. *European Polymer Journal*, 2012, vol. 48, 275-83
- **LAFRANCE, C P et al.** Study of the distribution of molecular orientation in highly oriented polyethylene by x-ray diffraction. *Macromolecules*, 1991, vol. 24, 4948-56
- **MOSELEY W W**. The measurement of molecular orientation in fibers by acoustic methods. *Journal of applied polymer science*, 1960, vol. 3, 266-76
- **BOULET-AUDET M et al.** Attenuated total reflection infrared spectroscopy: an efficient technique to quantitatively determine the orientation and conformation of proteins in single silk fibers. *Applied spectroscopy*, 2008, vol. 62, 956-62
- **ROUSSEAU M-E et al.** Study of protein conformation and orientation in silkworm and spider silk fibers using Raman microspectroscopy. *Biomacromolecules*, 2004, vol. 5, 2247-57
- **PEPPAS, N. A. et al.** Crystallization kinetics of poly(vinyl alcohol). *Journal of applied polymer science*, 1982, vol. 27, 4787-4797